# EUROPEAN PATENT APPLICATION

(11) **EP 4 389 047 A1**
(43) Date of publication of application: **26.06.2024**
(21) Application number: 23217667.7
(22) Date of filing: 18.12.2023
(51) Int. Cl.: A61B 34/10, A61B 34/20, A61B 1/00, A61B 90/94, A61B 90/00, A61B 17/00, G06N 20/00

(54) **SYSTEMS AND METHODS FOR GUIDING DRILLED HOLE PLACEMENT IN ENDOSCOPIC PROCEDURES**

(30) Priority: 19.12.2022 US 202263476158 P
(71) Applicant: Stryker Corporation, Kalamazoo, MI 49002 (US)
(72) Inventor: FOUTS, Brian, Kalamazoo, 49002 (US); HUNTER, Cole Kincaid, Kalamazoo, 49002 (US); GUREVICH, Lina, Kalamazoo, 49002 (US)
(74) Representative: V.O.

(57) **Abstract**

A method for visually indicating a location of a hole to be drilled in tissue includes, at a computing system: receiving at least one endoscopic image that captures a surface of the tissue and at least a portion of a drill guide to be used for drilling the hole in the tissue; identifying in the at least one endoscopic image at least one fiducial marker disposed on the drill guide; determining a position and orientation of the at least one fiducial marker; determining a position and orientation of the hole to be drilled in the tissue based at least in part on the position and orientation of the at least one fiducial marker; and displaying a visualization of the tissue that comprises a graphical indication of the position and orientation of the hole to be drilled in the tissue.

## Description

### CROSS-REFERENCE TO RELATED APPLICATIONS

This application claims the benefit of U.S. Provisional Application No. 63/476,158, filed December 19, 2022, the entire contents of which are hereby incorporated by reference herein.

### FIELD

This disclosure relates generally to endoscopic imaging, and more specifically, to generating visualizations from endoscopic imaging for providing visual guidance for drilling holes during an endoscopic procedure.

### BACKGROUND

Endoscopic imaging involves the use of a camera coupled to an endoscope inserted into a patient to provide a surgeon with a clear and precise view within the body. Video data collected by the camera is rendered on a display so that the surgeon can visualize the internal area of the body that is being viewed by the camera. The camera can serve as the eyes of the surgeon during the surgery since the camera may provide the only view of the internal area of the patient. In many instances, the surgeon may depend on the camera to perform procedures in the internal area of the patient using one or more tools that are specifically configured for endoscopic procedures to aid the surgeon as they perform the procedure. The surgeon can view the imaging feed being displayed to them during a surgery to manipulate the tool and navigate the tool within the internal area of the patient.

Orthopedics is a medical specialty involving the diagnosis, correction, prevention, and treatment of skeletal conditions, including conditions or disorders involving bones, joints, muscles, ligaments, and tendons. Orthopedic surgical procedures often involve manipulating bony anatomy. Such procedures may include, for example, the placement of anchors in bony anatomy. This often includes drilling a hole into the bony anatomy for insertion of a bone screw or other anchor. To aid in drilling the hole, surgeons may use a drill guide, which is typically positioned against a surface of the bony anatomy into which the hole is to be drilled. The drill guide includes a shaft for receiving and guiding the drill bit. The drill guide can assist in proper positioning and orienting of the drilled hole.

### SUMMARY

According to an aspect, systems and methods can provide visual guidance for the placement of a drilled hole in tissue during an endoscopic procedure. One or more endoscopic images of a surgical cavity that capture tissue into which a hole is to be drilled and a tool that includes at least one fiducial marker are analyzed to determine the position and orientation of the hole to be drilled in the tissue. A visualization is generated that includes one or more graphical indications of the position and orientation of the hole to be drilled, which may be overlaid on one or more endoscopic images. With this graphical guidance, a user may better understand how to position hole drilling tools so that the hole is drilled in a suitable location and with a suitable orientation.

A drill guide may be positioned in the surgical cavity and one or more fiducial markers of the drill guide captured in one or more endoscopic images can be used to determine the position and orientation of a hole that would be drilled using the drill guide. The known relationship between the fiducial marker(s) and the configuration of the drill guide can be used to determine the position and orientation of the hole that would be drilled using the drill guide. Alternatively or additionally, a pre-operative plan for where the hole should be drilled can be used to determine a position and orientation of a hole to be drilled in the tissue. One or more landmarks of the tissue captured in an endoscopic image can be identified and used to determine the position and orientation of the hole based on the pre-operative plan. One or more graphical indications of the position and orientation of the hole determined based on the position of the drill guide and/or based on the pre-operative plan can be displayed to the user, which can guide the user in positioning the drill guide for drilling the hole.

According to an aspect, a method for visually indicating a location of a hole to be drilled in tissue includes, at a computing system: receiving at least one endoscopic image that captures a surface of the tissue and at least a portion of a drill guide to be used for drilling the hole in the tissue; identifying in the at least one endoscopic image at least one fiducial marker disposed on the drill guide; determining a position and orientation of the at least one fiducial marker; determining a position and orientation of the hole to be drilled in the tissue based at least in part on the position and orientation of the at least one fiducial marker; and displaying a visualization of the tissue that comprises a graphical indication of the position and orientation of the hole to be drilled in the tissue. It will be appreciated that the endoscope and the drill guide may have been inserted into the subject prior to the start of the method. It will be appreciated that the endoscopic image is received at the computing system. The step of obtaining the endoscopic image is not part of the method. The visualization of the tissue that comprises the graphical indication of the position and orientation of the hole to be drilled in the tissue can be displayed to a surgeon to assist the surgeon in positioning and orienting the hole and/or the drill guide. Additionally, or alternatively, the displaying enables the surgeon to verify the correct position and orientation of the hole to be drilled and/or the drill guide. This may enable the surgeon to reposition the drill guide, if needed.

Optionally, the method includes determining an orientation of the surface of the tissue, wherein the position and orientation of the hole to be drilled in the tissue is determined based on the orientation of the surface of the tissue. The graphical indication may include a graphical representation of an estimated break-through in the surface of the tissue. A shape of the estimated break-through in the surface of the tissue may be based on the orientation of the hole to be drilled and the orientation of the surface of the tissue. The graphical representation of the estimated break-through in the surface of the tissue may have a diameter that corresponds to a diameter of a drill bit. The method may include receiving a user input corresponding to a selection of a different drill bit diameter and adjusting the diameter of the estimated break-through in the surface of the tissue based on the selection of the different drill bit diameter. The visualization may include a textual indication of the diameter of the drill bit.

The visualization may include a graphical representation of a shaft of the hole to be drilled. The tissue may include bony tissue.

The surface of the tissue in the at least one endoscopic image may be opposite a surface of the tissue where a drill bit will start drilling the hole to be drilled.

The visualization may include an indication of a distance from the hole to be drilled to a landmark of the tissue. The landmark of the tissue may be at a user-selected location of the tissue. The method may include receiving a user input relative to the displayed visualization selecting the user-selected location of the tissue. The landmark of the tissue is automatically identified in the at least one endoscopic image. The method may include receiving a user input selecting a different drill bit diameter and adjusting the indication of the distance from the hole to be drilled to the landmark of the tissue based on the different drill bit diameter.

Determining the orientation of the surface of the tissue may include generating a depth map that comprises depths at different locations of the surface of the tissue relative to a camera reference frame; and determining normal vectors of the surface of the tissue based on the depth map. Generating the depth map may include calibrating the depth map based on the position and orientation of the at least one fiducial marker. Generating the depth map may include predicting the depths at the different locations of the surface of the tissue using a machine learning model.

According to an aspect, a system includes one or more processors, memory, and at least one program stored in the memory for execution by the one or more processors and comprising instructions that, when executed by the one or more processors, cause the system to: receive at least one endoscopic image that captures a surface of the tissue and at least a portion of a drill guide to be used for drilling the hole in the tissue; identify in the at least one endoscopic image at least one fiducial marker disposed on the drill guide; determine a position and orientation of the at least one fiducial marker; determine a position and orientation of the hole to be drilled in the tissue based on the position and orientation of the at least one fiducial marker; and display a visualization of the tissue that comprises a graphical indication of the position and orientation of the hole to be drilled in the tissue.

The at least one program may include instructions for: determining an orientation of the surface of the tissue, wherein the position and orientation of the hole to be drilled in the tissue is determined based on the orientation of the surface of the tissue. The graphical indication may include a graphical representation of an estimated break-through in the surface of the tissue. A shape of the estimated break-through in the surface of the tissue may be based on the orientation of the hole to be drilled and the orientation of the surface of the tissue. The graphical representation of the estimated break-through in the surface of the tissue may have a diameter that corresponds to a diameter of a drill bit. The at least one program may include instructions for receiving a user input corresponding to a selection of a different drill bit diameter and adjusting the diameter of the estimated break-through in the surface of the tissue based on the selection of the different drill bit diameter. The visualization may include a textual indication of the diameter of the drill bit.

The visualization may include a graphical representation of a shaft of the hole to be drilled. The tissue may include bony tissue.

The surface of the tissue in the at least one endoscopic image may be opposite a surface of the tissue where a drill bit will start drilling the hole to be drilled.

The visualization may include an indication of a distance from the hole to be drilled to a landmark of the tissue. The landmark of the tissue may be at a user-selected location of the tissue. The at least one program may include instructions for receiving a user input relative to the displayed visualization selecting the user-selected location of the tissue. The landmark of the tissue may be automatically identified in the at least one endoscopic image. The at least one program may include instructions for receiving a user input selecting a different drill bit diameter and adjusting the indication of the distance from the hole to be drilled to the landmark of the tissue based on the different drill bit diameter.

Determining the orientation of the surface of the tissue may include: generating a depth map that comprises depths at different locations of the surface of the tissue relative to a camera reference frame; and determining normal vectors of the surface of the tissue based on the depth map. Generating the depth map may include calibrating the depth map based on the position and orientation of the at least one fiducial marker. Generating the depth map may include predicting the depths at the different locations of the surface of the tissue using a machine learning model.

According to an aspect, a method for visually indicating a location of a hole to be drilled in tissue includes, at a computing system: receiving at least one endoscopic image that captures a surface of the tissue and at least a portion of a tool; identifying in the at least one endoscopic image and at least one fiducial marker disposed on the tool; determining one or more characteristics of the at least one fiducial marker; determining position information for the surface of the tissue based on the one or more characteristics of the at least one fiducial marker; determining a position of at least one landmark of the tissue based on the position information for the surface of the tissue; determining a position and orientation of the hole to be drilled in the tissue based on the position of the at least one landmark and a pre-operative plan; and displaying a visualization of the tissue that comprises a graphical indication of the position and orientation of the hole to be drilled in the tissue. It will be appreciated that the endoscope and the tool may have been inserted into the subject prior to the start of the method. It will be appreciated that the endoscopic image is received at the computing system. The step of obtaining the endoscopic image is not part of the method. The visualization of the tissue that comprises the graphical indication of the position and orientation of the hole to be drilled in the tissue can be displayed to a surgeon to assist the surgeon in positioning and orienting the hole and/or the tool. Additionally, or alternatively, the displaying enables the surgeon to verify the correct position and orientation of the hole to be drilled and/or the tool. This may enable the surgeon to reposition the tool, if needed.

The method may include determining position information for the surface of the tissue comprises generating a depth map that comprises depths at different locations of the surface of the tissue relative to a camera reference frame and calibrating the depth map using the one or more characteristics of the at least one fiducial marker. Determining the position of the at least one landmark may include determining a depth of the at least one landmark based on the depth map. Determining the position of the at least one landmark may include automatically identifying the at least one landmark. The method may include, prior to determining the position of the at least one landmark, receiving a user input selecting the at least one landmark.

According to an aspect, a system includes one or more processors, memory, and at least one program stored in the memory for execution by the one or more processors and comprising instructions that, when executed by the one or more processors, cause the system to: receive at least one endoscopic image that captures a surface of the tissue and at least a portion of a tool; identify in the at least one endoscopic image and at least one fiducial marker disposed on the tool; determine one or more characteristics of the at least one fiducial marker; determine position information for the surface of the tissue based on the one or more characteristics of the at least one fiducial marker; determine a position of at least one landmark of the tissue based on the position information for the surface of the tissue; determine a position and orientation of the hole to be drilled in the tissue based on the position of the at least one landmark and a pre-operative plan; and display a visualization of the tissue that comprises a graphical indication of the position and orientation of the hole to be drilled in the tissue.

Determining position information for the surface of the tissue may include generating a depth map that comprises depths at different locations of the surface of the tissue relative to a camera reference frame and calibrating the depth map using the one or more characteristics of the at least one fiducial marker. Determining the position of the at least one landmark may include determining a depth of the at least one landmark based on the depth map. Determining the position of the at least one landmark may include automatically identifying the at least one landmark.

The at least one program may include instructions for, prior to determining the position of the at least one landmark, receiving a user input selecting the at least one landmark.

According to an aspect, a non-transitory computer readable storage medium stores instructions for execution by a computing system to cause the computing system to perform any of the methods above.

It will be appreciated that any of the variations, aspects, features and options described in view of the systems apply equally to the methods and vice versa. It will also be clear that any one or more of the above variations, aspects, features and options can be combined.

### BRIEF DESCRIPTION OF THE FIGURES

The invention will now be described, by way of example only, with reference to the accompanying drawings, in which:
FIG. 1 illustrates an exemplary endoscopy system;
FIG. 2A and FIG. 2B illustrate aspects of an exemplary ligament reconstruction procedure in a knee joint in which hole placement guidance can be provided;
FIG. 3 is an example of a visualization providing one or more graphical indications of the placement of a hole in tissue;
FIG. 4 is a diagram of a method for visually indicating a location of a hole to be drilled in tissue;
FIG. 5 is a diagram of a method for determining the orientation of a surface where a hole is to be drilled;
FIG. 6 provides an illustration of the values of an exemplary depth map generated from an endoscopic image;
FIG. 7A-7C illustrate examples of rotations of a circle that may be used to provide a graphical indication of a hole to be drilled on a surface of tissue;
FIG. 8 illustrates an exemplary method for generating and displaying a visualization that includes a graphical indication of the position and orientation of a hole to be drilled in tissue that is generated based on a pre-operative plan;
FIG. 9 illustrates an example of an endoscopic image that captures tissue of interest into which a hole is to be drilled and a tool that includes one or more fiducial markers;
FIG. 10 illustrates an exemplary visualization of tissue of interest in which one or more graphical indications of one or more holes drilled in the tissue are relocated in the visualization based on movement of the endoscopic imager such that the graphical indication(s) continues to accurately indicate the position of the hole(s) relative to the displayed scene;
FIG. 11 illustrates an exemplary method for repositioning a graphical indication of a hole drilled in tissue in a visualization according to movement of an endoscopic imager;
FIG. 12 illustrates an example of a computing system;
FIG. 13 illustrates an example of how a distance from the hole to a location of interest may be calculated;
FIGS. 14A-14D illustrate an example of how the orientation of the surface of the tissue and the orientation of the hole to be drilled can be used in combination to determine the shape of a graphical indication of the hole to be drilled;
FIG. 15 is a flow diagram of an exemplary method for estimating motion of an endoscopic imager between frames;
FIG. 16 illustrates an exemplary key frame cloud used for tracking motion of an endoscopic imager; and
FIG. 17 is a flow diagram of an exemplary method for building a key frame cloud.

### DETAILED DESCRIPTION

Reference will now be made in detail to implementations and embodiments of various aspects and variations of systems and methods described herein. Although several exemplary variations of the systems and methods are described herein, other variations of the systems and methods may include aspects of the systems and methods described herein combined in any suitable manner having combinations of all or some of the aspects described.

Systems and methods described herein can provide visual guidance for the placement of a drilled hole in tissue of a subject during an endoscopic procedure. Endoscopic images that capture tissue of interest and one or more fiducial markers on a tool are analyzed to determine the position and orientation of a hole to be drilled in the tissue. A visualization can be generated that includes one or more graphical indications of the position and orientation of the hole overlaid on the one or more endoscopic images so that the user can visualize where the hole will be and how it will be oriented relative to the point of view in the endoscopic images. The visualization can be displayed to a surgeon to assist the surgeon in positioning and orienting the hole and/or the tool. Additionally, or alternatively, the displaying enables the surgeon to verify the correct position and orientation of the hole to be drilled and/or the tool. This may enable the surgeon to reposition the tool, if needed.

The one or more graphical indications can include a graphical representation of a break-through of the hole in the surface of the tissue. The graphical representation of a break-through can be shaped based on the orientation of the hole to be drilled. The graphical representation of a break-through can be shaped based also on the orientation of the surface of the tissue into which the hole is to be drilled. The orientation of the surface of the tissue can be determined by generating a depth map that provides depths of the tissue in the endoscopic images and determining surface normals based on rates of change of the depths. The shape of a graphical representation of a break-through in the surface of the tissue can be generated by rotating a circle in three-dimensional space based on the orientation of a surface normal for the tissue at the position where the hole is to be drilled and based on the orientation of the hole to be drilled.

The position and orientation of the hole to be drilled can be determined based on the position and orientation of a drill guide upon which the fiducial marker is positioned. The predetermined position and orientation of the fiducial marker on the drill guide can be used in combination with the position and orientation of the fiducial marker in the endoscopic images to determine where the hole will be positioned and how the hole will be oriented with respect to the endoscopic images, which can be used to generate an overlay of one or more graphical indications of the position and orientation of the hole on the endoscopic images.

The position and orientation of the hole to be drilled can be determined based on one or more landmarks of the tissue that are captured in the endoscopic images and a pre-operative plan for where the hole is to be placed relative to those landmarks. The one or more landmarks can be automatically identified or can be selected by a user. The positions of the landmarks and of the tissue where the hole is to be drilled can be determined by generating a depth map from the endoscopic images and calibrating the depth map using a fiducial marker on a tool that is captured in the endoscopic images. Once the positions of the landmarks are determined, the position and orientation of the hole to be drilled can be determined based on the planned position and orientation of the hole relative to the landmarks in the pre-operative plan. Visual guidance for the planned position and orientation of the hole can be displayed to the user to assist the user in positioning a drill guide for drilling the hole.

The systems and methods described herein can lead to improved surgical outcomes by providing visual guidance for the proper placement of drilled holes during endoscopic procedures. The visual guidance can reduce the amount of guesswork used in determining where to drill a hole, leading to more consistent hole placement. More consistent hole placement can lead to more consistent surgical outcomes for procedures in which hole placement is important, such as anterior cruciate ligament (ACL) and posterior cruciate ligament (PCL) reconstruction surgeries.

In the following description of the various embodiments, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating" or the like, refer to the action and processes of a computer system, or similar electronic computing device, that manipulates and transforms data represented as physical (electronic) quantities within the computer system memories or registers or other such information storage, transmission, or display devices.

The present disclosure in some embodiments also relates to a device for performing the operations herein. This device may be specially constructed for the required purposes, or it may comprise a general purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each connected to a computer system bus. Furthermore, the computing systems referred to in the specification may include a single processor or may be architectures employing multiple processor designs, such as for performing different functions or for increased computing capability. Suitable processors include central processing units (CPUs), graphical processing units (GPUs), field programmable gate arrays (FPGAs), and ASICs.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present disclosure as described herein.

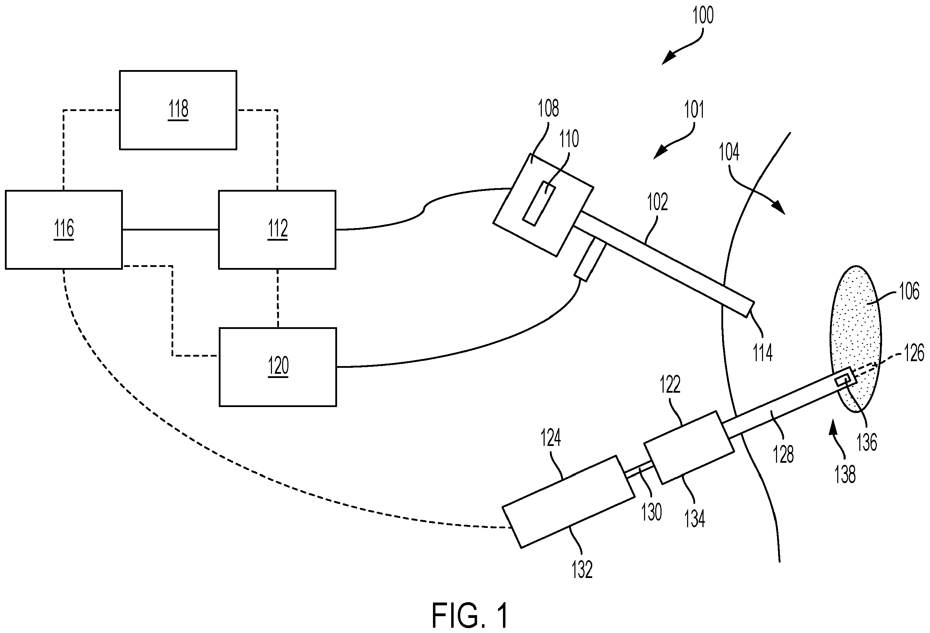

FIG. 1 illustrates an exemplary endoscopy system 100. System 100 includes an endoscopic imager 101 that can include a camera head 108 mounted to an endoscope 102. As is well-known in the art, the endoscope 102 can be configured for insertion into a surgical cavity 104 for imaging tissue 106 within the surgical cavity 104 during a medical procedure. Light generated by a light source 120 may be directed through the endoscope 102 to the surgical cavity 104. Light reflected by and/or emitted from the tissue 106 (such as fluorescence light emitted from fluorescing targets that are excited by fluorescence excitation illumination light provided by the light source 120) is received at the distal end 114 of the endoscope 102. The light is propagated by the endoscope 102, such as via one or more optical components (for example, one or more lenses, prisms, light pipes, or other optical components), to the camera head 108, where it is directed onto one or more imaging sensors 110. One or more filters (not shown) may be included in the endoscope 102 in a coupler (not shown) connecting the endoscope 102 to the camera head 108, and/or in the camera head 108 for filtering a portion of the light received from the tissue 106 (such as fluorescence excitation light used for fluorescence imaging).

The one or more imaging sensors 110 generate pixel data that can be transmitted to a camera control unit 112 that is communicatively connected to the camera head 108. The camera control unit 112 can generate endoscopic images (as used herein, "endoscopic image(s)" encompasses single snapshot images and/or a sequence of video frames) from the pixel data that shows the tissue being viewed by the endoscopic imager 101. The endoscopic images can be transmitted to an image processing system 116 for further image processing, storage, display, and/or routing to an external device (not shown). The image processing system 116 receives the endoscopic images from the camera control unit 112. The endoscopic images can be transmitted to one or more displays 118, from the camera control unit 112 and/or the image processing system 116, for visualization by medical personnel, such as by a surgeon for visualizing the surgical cavity 104 during a surgical procedure on a patient. The camera control unit 112 and/or the image processing system 116 may be configured to send control signals to the light source 120 and/or the camera head 108 to control one or more aspects of the imaging, such as a timing sequence of light provided by the light source 120 (e.g., a sequence of white light and fluorescence excitation light), an amount of light provided by the light source 120, and/or a gain of the one or more imaging sensors 110.

One or more surgical tools may be used in the surgical cavity 104 to manipulate tissue 106 during a surgical procedure on the patient, and the surgical tools may be captured in the images captured by the camera head 108 and displayed on the display 118 so that the medical personnel can view the interaction between the one or more surgical tools and the tissue. For example, the tissue 106 may be bone, and the one or more tools may include a drill guide 122 used for guiding a drill 124 to drill a hole 126 in the bone. As is known in the art, a surgeon may position the drill guide 122 against the tissue 106 at a desired position and may orient the drill guide 122 according to a desired orientation for the hole 126. The drill guide 122 includes a shaft 128 for receiving and guiding a drill bit 130 of the drill 124. The surgeon may activate the drill 124 and advance the drill bit 130 into the tissue 106 to drill the hole 126. The surgeon may advance the drill bit 130 until a proximal portion 132 of the drill 124 abuts a proximal portion 134 of the drill guide 122, which stops advancement of the drill bit 130 within the tissue 106, thereby controlling the depth advancement of the drill bit 130 in the tissue 106 (for example, a depth of the hole 126). The drill 124 may be communicatively connected to the image processing system 116, which may enable a user to indicate a desire for the image processing system 116 to predict a position of the hole 126 prior to the hole 126 being drilled.

The location and/or orientation of the hole 126 in the tissue 106 may be important for the success of the procedure. With the limited field of view provided by endoscopic imaging, it may be difficult for a surgeon to know the precise location and/or orientation of the hole 126 prior to the hole 126 being drilled. As described in detail below, to guide a surgeon in determining placement of the hole 126, the system 100 may analyze one or more endoscopic images to predict where a hole will be placed based on the position and orientation of a drill guide and may generate and display a visualization (e.g., on display 118) that includes one or more graphical indications of the predicted placement of the hole 126 in a displayed endoscopic image.

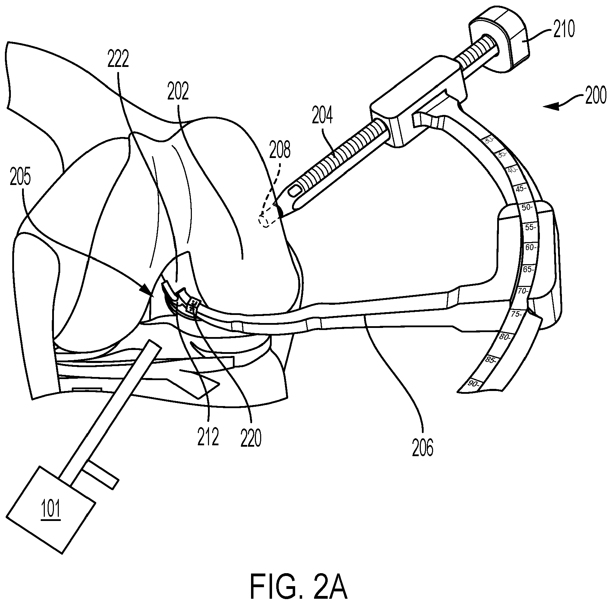

FIG. 2A illustrates aspects of an exemplary ligament reconstruction procedure in a knee joint in which hole placement guidance provided by system 100 may be particularly useful. The ligament reconstruction procedure may be, for example, an ACL graft or a PCL graft. As is known in the art, an ACL reconstruction procedure may involve the drilling of holes in the femur and tibia and the threading of a ligament graft through the tibial and femoral holes. The placement of the holes in the respective bones can be important to the ultimate success of the procedure. The placement of the hole may be important to the kinematics of the j oint, such as to the ultimate range of motion of the joint and/or to the tightness of the joint. In some cases, improper placement of the hole too close to a surface of the bone may lead to bone fracture due to insufficient wall thickness of the bone to support the grafted ligament. A visualization generated by system 100 that provides a graphical indication of the predicted placement of the hole relative in a displayed endoscopic image can enable a surgeon to locate an ideal placement of the hole, which can lead to improved surgical outcomes.

The phase of the ligament reconstruction procedure shown in FIG. 2A involves the drilling of a hole (also referred to as a tunnel) through the femoral condyle 202 using an "outside-in" technique with the aid of a drill guide 200. The drill guide 200 includes a guide shaft 204 for guiding a drill bit and an arm 206 that extends from the guide shaft 204. The distal end 208 of the guide shaft 204 is positioned against the lateral side of the femoral condyle 202. A drill bit is inserted into the proximal end 210 of the guide shaft 204 and drills through the femoral condyle 202 from the outside-in. The distal end 212 of the arm 206 is aligned with the guide shaft 204 and shaped to indicate to the surgeon where the drill bit will emerge from the femoral condyle 202 within the joint space 205. The distal end 212 can be circular or arcuate in shape and aligned with the guide shaft 204 such that the drill bit will emerge centrally within the space defined by the circular or arcuate distal end 212.

An endoscopic imager 101 is positioned within the j oint space 205 such that the location where the hole in the femoral condyle 202 is to be drilled can be viewed in endoscopic images generated using the endoscopic imager 101. The surgeon positions the distal end 212 of the arm 206 against the surface 222 of the femoral condyle 202 and adjusts its position so that the distal end 212 is positioned at a location on the surface 222 of the femoral condyle 202 where the surgeon desires the hole to emerge. As such, the surgeon visualizes in the endoscopic images the surface of the tissue where the hole will emerge, which is opposite the surface of the tissue where the drill bit will start drilling the hole.

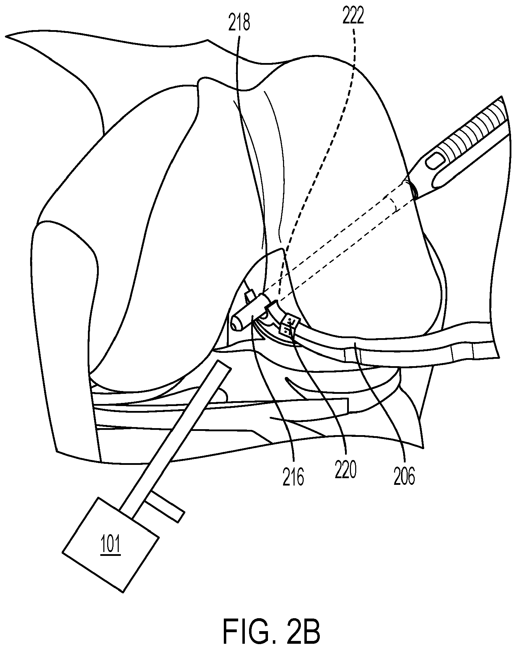

FIG. 2B shows a drill bit 216 that has drilled a hole 218 through the femoral condyle 202 from the outside-in. The drill bit 216 has broken through the femoral condyle 202 and is located centrally within the arcuate distal end 212 of the arm 206.

As noted above, the placement of the hole 218 can be important in the success of the ligament reconstruction procedure shown in FIGS. 2A and 2B. Although the distal end 212 of the arm 206 of the drill guide 200 indicates where the hole 218 will emerge on the surface 222 of the femoral condyle 202, it may be difficult for the surgeon to know exactly where the hole 218 will be and what orientation the hole 218 will have once drilled. To guide a surgeon in visualizing the position and orientation of the hole prior to the hole being drilled, system 100 can analyze one or more endoscopic images to determine the position and orientation of the hole based on the position and orientation of the drill guide 200. The arm 206 may include one or more fiducial markers 220 located at or near the distal end 212 of the arm 206 such that the one or more fiducial markers 220 are within the field of view of the endoscopic imager 101. The system 100 identifies the one or more fiducial markers 220 in one or more endoscopic images. The system 100 determines the position and orientation of the drill guide based on the position and orientation of the one or more fiducial markers 220 and the predetermined relationship between the position and orientation of the one or more fiducial markers 220 and the position and orientation of one or more features of the drill guide 200. The system 100 then generates a visualization that provides one or more graphical indications of where a hole will be positioned after being drilled by a drill bit that was guided by the drill guide.

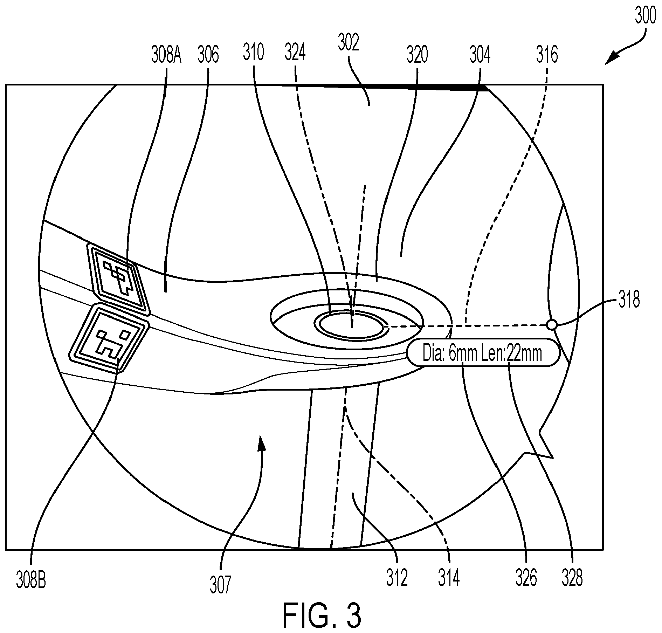

FIG. 3 is an example of a visualization providing one or more graphical indications of the placement of a hole in tissue that may be generated by system 100. The visualization 300 includes an endoscopic image 302 that captures tissue 304 into which a hole is to be drilled and a distal portion 307 of an arm 306 of a drill guide. The arm 306 includes fiducial markers 308A, 308B. System 100 receives the endoscopic image 302. System 100 analyzes the endoscopic image 302 to identify at least one of the fiducial markers 308A, 308B. System 100 determines the position and orientation of the at least one of the fiducial markers 308A, 308B. System 100 determines the position and orientation of a hole that would be drilled by a drill bit guided by the drill guide based on predetermined relationships between the position and orientation of the at least one of the fiducial markers and the position and orientation of one or more features of the drill guide (e.g., predetermined relationship between the position and orientation of fiducial marker 308A and the position and orientation of distal end 320 of the arm 306 of the drill guide). Based on the determined position and orientation of the hole that would be drilled by a drill bit guided by the drill guide, the system 100 generates the visualization 300 by overlaying onto the endoscopic image 302 one or more graphical indications of where the hole would be drilled. The graphical indications can include, for example, a graphical representation 310 of an estimated breakthrough of the hole through the surface of the tissue 304, a graphical representation 312 of the shaft of the hole illustrating the trajectory of the hole within the tissue 304, and/or a graphical representation 314 of a longitudinal axis of the hole, which also illustrates the trajectory of the hole.

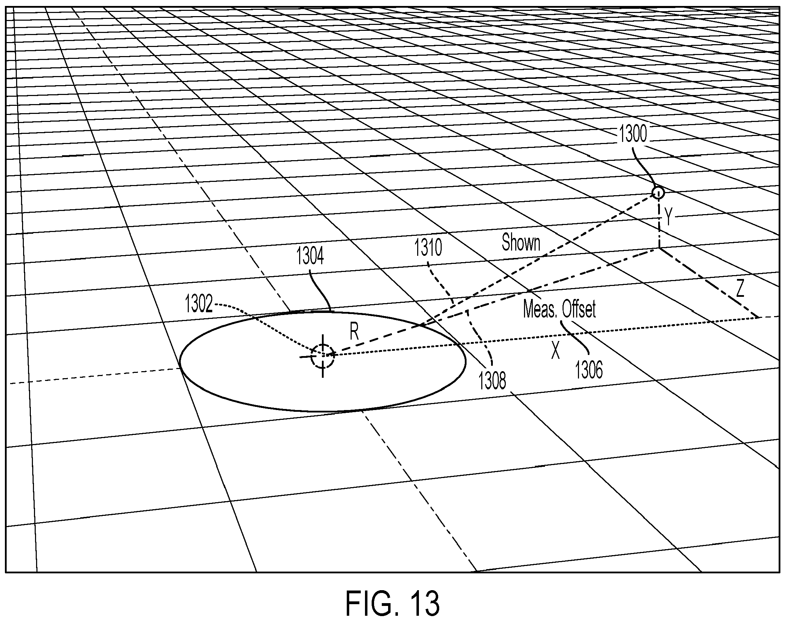

The visualization 300 can also include a graphical indication 316 of a distance from the hole (e.g., the perimeter of the hole and/or radial offset) to a location of interest 318, such as an anatomical landmark, or a location associated with the location of interest (such as a projection of a location of interest onto a plane containing the perimeter of the hole to be drilled, as described below). This may be useful, for example, in indicating whether the hole is too close to or too far from a location of interest, such as a back wall of a bone (and, therefore, whether the bone wall thickness may be too thin). FIG. 13 illustrates an example of how a distance from the hole to be drilled to a location of interest or a location associated with the location of interest may be calculated. A three-dimensional position of a location of interest 1300 (e.g., selected by the user or automatically identified) is determined based on a coordinate system in which a center 1302 of a hole to be drilled is the origin and the perimeter 1304 of the hole to be drilled lies in the plane containing two of the axes of the coordinate system (e.g., the -xz plane as in the illustrated example). Next, the components of the three-dimensional position of the location of interest 1300 that are in the plane containing the perimeter of the hole to be drilled (e.g., the -x and -z components in the illustrated example) are used to calculate the distance 1306 to the perimeter 1304 of the hole to be drilled. This distance 1306 can then be displayed to the user.

A graphical indication 1308 of the line segment defining the distance 1306 can be shown. Alternatively, since the graphical indication 1308 may not connect to the location of interest 1300 (it will connect to the location of interest 1300 in instances where the location of interest 1300 and the perimeter 1304 of the hole to be drilled lie in the same plane) which may be confusing to the user, a graphical indication 1310 can be shown that connects the perimeter 1304 with the location of interest 1300.

Visualization 300 is merely exemplary of the graphical indications that may be provided for indicating the predicted position and orientation of the hole. Visualizations may be generated that have one or more of the graphical indications shown in FIG. 3 and/or other graphical indications associated with the predicted placement of the hole.

The distance 1306 from the location of interest 1300 to the perimeter 1304 of the hole to be drilled can be updated over time after the user has moved the drill guide. This distance 1306 can be monitored while the user is drilling the hole. Should the distance 1306 drop below a predetermined threshold as the hole is being drilled (e.g., due to the drill guide moving relative to the tissue), the user may be provided with a warning. Optionally, the image processing system 116 may additionally or alternatively control the drill to stop the drilling when the distance 1306 drops below the threshold, such as to prevent the hole from being drilled too close to a wall of the tissue.

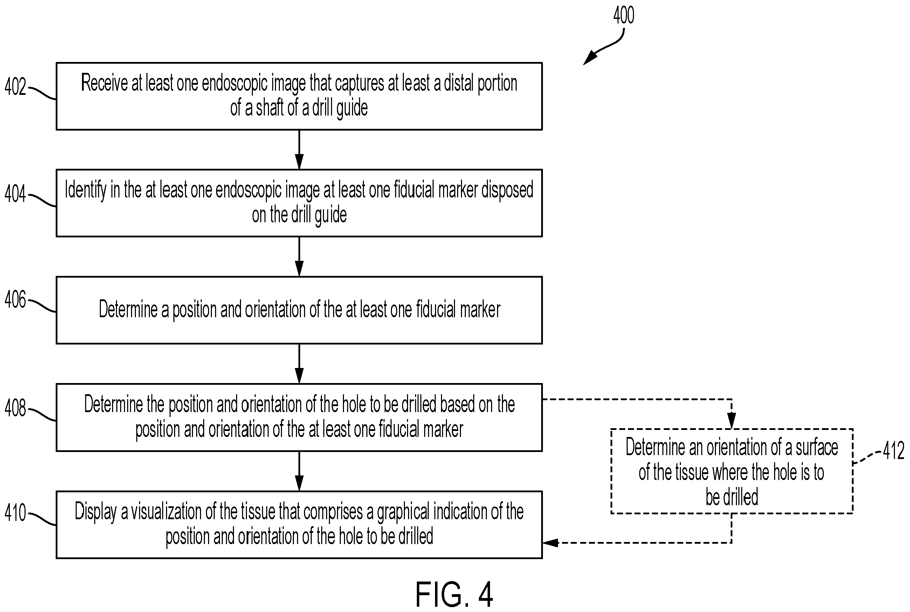

FIG. 4 is a diagram of a method 400 for visually indicating a location of a hole to be drilled in tissue. Method 400 can be used for generating visualization 300 of FIG. 3. Method 400 can be performed by an image processing system, such as image processing system 116 of system 100 of FIG. 1. Method 400 can be performed during a surgical procedure on a subject to provide guidance to one or more users (e.g., surgeons, medical staff, and the like) during the surgical procedure.

At step 402, at least one endoscopic image is received at an image processing system. For example, with respect to FIG. 1, one or more endoscopic images (e.g., a single snapshot image and/or a sequence of video frames) generated by camera control unit 112 based on pixel data from the endoscopic imager 101 may be received by image processing system 116. The endoscopic imager can be pre-inserted prior to start of method 400. The endoscopic image(s) capture tissue of interest within a surgical cavity, such as tissue 106 within surgical cavity 104 of FIG. 1. The tissue of interest may be, for example, bony anatomy. The tissue of interest may be tissue into which a hole will be drilled. The endoscopic image(s) may also capture a distal portion of a drill guide that is located in the surgical cavity. The drill guide can be pre-inserted prior to start of method 400. For example, with respect to FIG. 1, a distal portion 138 of drill guide 122 may be located in the surgical cavity 104 within a field of view of the endoscopic imager 101. The distal end of the drill guide may be positioned against the tissue of interest, such as for guiding a drill bit to drill a hole in a desired position of the tissue of interest. For example, with respect to FIG. 2A, the distal end 212 of the arm 206 of the drill guide 200 may be positioned against the surface 222 of the femoral condyle 202.

One or more fiducial markers located at the distal portion of the drill guide may be captured in the endoscopic image(s). For example, with respect to FIG. 1, fiducial marker 136 may be within the field of view of endoscopic imager 101 such that the fiducial marker 136 is captured in endoscopic image(s) generated by the endoscopic imager 101. More than one fiducial marker may be captured in the endoscopic image(s). For example, as shown in FIG. 3, the multiple fiducial markers 308A, 308B may be captured in the endoscopic image 302. The at least one endoscopic image may be a series of frames of a video feed. The steps described below may be performed on just one frame of the video feed, on each frame of the video feed, or on a subset of the frames of the video feed.

At step 404, at least one fiducial marker of the drill guide is identified in the at least one endoscopic image. The fiducial marker can be identified by detecting one or more visual patterns of the fiducial marker and matching the one or more visual patterns to at least one fiducial marker pattern in a pre-defined list of fiducial marker patterns. The one or more visual patterns can be detected according to well-known image processing techniques, such as using a suitable edge detection algorithm and searching for edges that correspond to features of pattern and/or using a machine learning model trained to identify one or more fiducial markers in endoscopic images. Identification of the fiducial marker in step 404 may include extraction of information encoded by the fiducial marker. For example, the fiducial marker may be an ArUco marker that encodes an identity of the ArUco marker that may uniquely identify the ArUco marker relative to other ArUco markers such that the image processing system can access the predetermined spatial information associated with that ArUco marker (or the feature(s) of the drill guide associated with that ArUco marker). The ArUco marker may additionally comprise encoded error detection and correction information such that any discrepancies and/or errors in detecting the bit pattern of the ArUco marker may be minimized to determine the correct information associated with a given ArUco marker. Other examples of fiducial markers that may be used are bar codes, Quick Response (QR) codes, and AprilTags.

One or more machine learning models can be used to improve the accuracy of finding and identifying the fiducial markers with respect step 404. For example, a machine learning model can be used to segment the drill guide in the endoscopic image. By segmenting the endoscopic image (i.e., determining the region of the endoscopic image that includes the drill guide), an image processing algorithm can focus on the segmented region to search for the fiducial marker so that the fiducial marker can be more easily found than if the entire image were searched. In one or more examples, a sequence of endoscopic images (a sequence of endoscopic video frames) can be analyzed by one or more machine learning models so that, for instance, any temporal aspects of the video can be used by the one or more machine learning models to segment the tool from an image and/or otherwise help to locate and identify the fiducial markers. In one or more examples, the one or more machine learning models can be an instance segmentation machine learning model configured to detect, segment, and classify individual objects in an image. An example of a suitable instance segmentation machine learning model that works on individual images is a Mask R-CNN. Examples of suitable instance segmentation machine learning model that work on video input and utilizes the temporal component of the video is a multi-scale spatio-temporal split (MS-STS) transformer and spatial-temporal graph neural networks.

At step 406, a position and orientation of at least a portion of the at least one fiducial marker in the at least one endoscopic image is determined. As is well-known in the art, predetermined information regarding the positions of features of a fiducial marker relative to one another in a world reference frame can be used to calibrate the positions of the features of the fiducial marker in the endoscopic image (e.g., the pixel positions) such that the position and orientation of the fiducial marker (or portions of the fiducial marker) in a suitable reference frame, such as a camera-based reference frame, can be determined.

With reference to system 100 of FIG. 1, the predetermined information regarding the fiducial marker may be stored in a memory of the image processing system 116 or may be stored in a memory of a computing device to which the image processing system 116 is communicatively connected, such as a memory of a hospital information network or a cloud-based storage. The predetermined information can be, for example, measurements of spacing between different features of the fiducial marker (e.g., spacing between corners of the fiducial marker) stored in a look-up table (LUT). The predetermined information regarding the fiducial marker may be retrieved from the memory by the image processing system based on information encoded in the fiducial marker. For example, a fiducial marker identification encoded in the fiducial marker may be cross referenced in a fiducial marker database to access the predetermined fiducial marker information associated with that particular fiducial marker.

At step 408, the position and orientation in an endoscopic image of a hole to be drilled in tissue by a drill bit guided by the drill guide is determined based on the position and orientation of the at least one fiducial marker in the endoscopic image determined in step 406. The position and orientation of the hole to be drilled can be determined, for example, by determining the position and orientation of a feature of the drill guide and using the position and orientation of the feature as the position and orientation of the hole to be drilled. For example, with respect to FIG. 3, the position of the hole to be drilled in the endoscopic image 302 can be determined by determining the position of the center 324 of the distal end 320 of the arm 306 of the drill guide in the endoscopic image 302, which may be used as the position of the hole to be drilled (e.g., the position of the entrance to the hole) in the endoscopic image. The orientation of the hole to be drilled can be determined by determining orientation of the distal end 320 of the arm 306.

The position and orientation of the feature of the drill guide used to determine the position and orientation of the hole to be drilled can be determined based on predetermined information associated with the location of the feature relative to the one or more fiducial markers of the drill guide. For example, the position of the center 324 of the distal end 320 of the drill guide can be determined based on the position and orientation of one or more of the fiducial markers 308A, 308B identified in the endoscopic image 302 and the predetermined position of the center 324 of the distal end 320 relative to those fiducial markers 308A, 308B or relative to one or more portions of those fiducial markers 308A, 308B that are stored in memory and accessed by the image processing system. The orientation of the drill guide or a feature of the drill guide determined based on the position and orientation of the one or more fiducial markers can be used, for example, to determine the orientation of the hole to be drilled. The orientation of the drill guide or a feature of the drill guide may be, for example, a set of rotations about axes of a reference frame, such as rotations about x-, y-, and z-axes of a camera-based reference frame.

The information associated with the location of the feature relative to the one or more fiducial markers of the drill guide may be stored in a memory of the image processing system performing method 400 (e.g., image processing system 116) or may be stored in a memory of a computing device to which the image processing system is communicatively connected, such as a memory of a hospital information network or a cloud-based storage. The predetermined information can be, for example, measurements of spacing between the feature of the drill guide and one or more fiducial markers or one or more portions of one or more fiducial markers.

At step 410, a visualization of the tissue is displayed (e.g., on display 118 of system 100 of FIG. 1) that includes at least one graphical indication of the position and orientation of the hole to be drilled in the tissue. The visualization can include an endoscopic image with one or more graphical indications indicating the predicted position and orientation of the hole to be drilled overlayed on the endoscopic image, such as one or more of graphical representations 310, 312, and 314 of visualization 300 of FIG. 3.

The position and orientation of the hole to be drilled determined in step 408 can be used to determine the position and shape of the graphical indication(s) displayed in the visualization. For example, the graphical indication of the hole to be drilled may be an oval indicating a perimeter of the hole to be drilled. The shape of the oval may be determined by centering a circle at the center of the hole to be drilled as determined in step 408, scaling the circle in diameter according to the position of the hole to be drilled relative to the camera as determined in step 408 and a predetermined diameter of the hole (e.g., associated with a diameter of a drill bit to be used to drill the hole), and rotating the circle in three-dimensional space according to the predetermined orientation of the distal end of the drill guide relative to the orientation of the hole to be drilled as determined in step 408.

The visualization displayed in step 410 may be user modifiable via one or more user input features, such as one or more graphical selectors of a graphical user interface, one or more physical buttons (e.g., on the image processing system 116, on the camera head 108, and/or on the drill 124), a voice command system, etc. For example, the position of the graphical indication of the hole to be drilled may be user adjustable, which could be useful in scenarios in which the prediction of the hole position was not accurate. The user may be able to select to display or hide the graphical indication of the hole to be drilled.

A user may be able to adjust a size of the graphical indication of the hole to be drilled, which may be useful for the user to assess the effect of different drill bit sizes on the hole. For example, a user may provide an input selecting a predetermined drill bit type and/or size (e.g., diameter) and the size (e.g., a diameter of graphical indication 310) of a visual indication of a perimeter of the hole to be drilled may be adjusted according to a predetermined size of the selected drill bit type and/or size. The user input selecting the predetermined drill bit type and/or size can be provided in any suitable fashion, such as via an input associated with a graphical selector in a graphical user interface, an input to a user input device of the image processing system (e.g., a button or a touch screen of the image processing system), a voice command, and/or a press of a button of a camera head of an endoscopic imager. The visualization generated at step 410 of method 400 can include an indication of the selected drill bit type and/or size. For example, the visualization 300 of FIG. 3 provides the diameter 326 (6 mm) in textual format of the selected drill bit type and/or size.

The determination of the position of a hole to be drilled in an endoscopic image according to method 400 may be triggered manually by a user, such as via a user input. As such, the image processing system, such as image processing system 116 of system 100, may perform one or more steps of method 400 in response to a user command to provide a graphical indication of the hole to be drilled. The user command can be provided in any suitable fashion, such as via an input associated with a graphical selector in a graphical user interface, an input to a user input device of the image processing system (e.g., a button or a touch screen of the image processing system), a voice command, and/or a press of a button of a camera head of an endoscopic imager. Optionally, the drill used to drill the hole may be communicatively connected to an image processing system (e.g., as shown in FIG. 1) and may include a user input device, such as a button, that enables the user to command hole detection by the image processing system.

The graphical indication of the hole to be drilled may be generated based not only on the position and orientation of the drill guide but also based on an orientation of the surface of the tissue where the hole will be drilled, which can provide a more accurate indication to the user of where the hole will be positioned. For example, the shape of the graphical indication may be based on the orientation of the drill guide in relation to the orientation of the surface of the tissue at the location where the hole will be drilled, such that the graphical indication is a graphical representation of an estimated break-through of the hole in the surface of the tissue. For example, with reference to visualization 300 of FIG. 3, the shape of the graphical representation 310 may be based on the orientation of the tissue 304 captured in the endoscopic image 302 in relation to the orientation of the drill guide. Accordingly, method 400 can include an optional step 412 in which the orientation of the surface of the tissue where the hole is to be drilled is determined. The orientation of the surface of the tissue where the hole is to be drilled can be used in step 410 to determine the shape of one or more graphical indications of the position and orientation of the hole to be drilled.

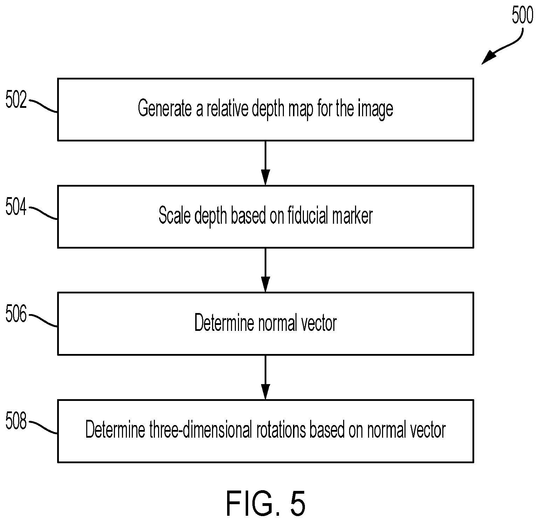

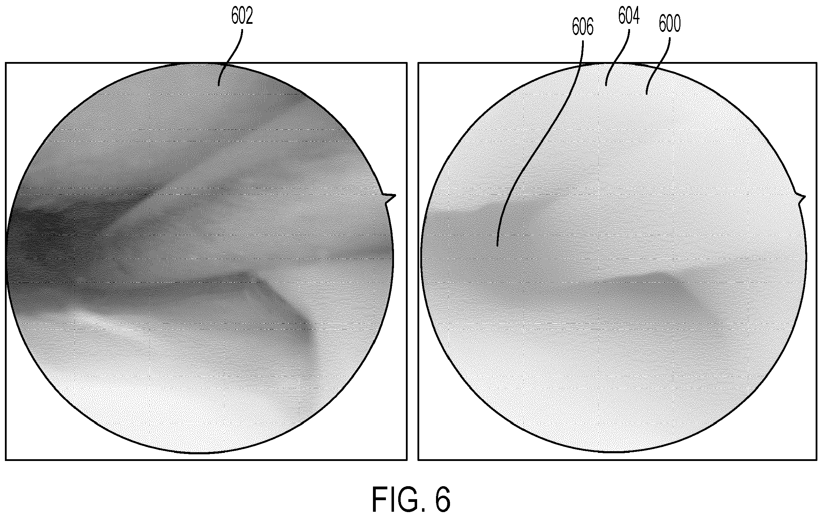

FIG. 5 is a diagram of a method 500 for determining the orientation of a surface where a hole is to be drilled that may be used for step 412 of method 400. At step 502, a relative depth map is generated for an endoscopic image. The relative depth map may be generated for the same endoscopic image used for determining the position and orientation of the hole to be drilled or may be determined for one or more other endoscopic images. The relative depth map may be generated by a machine learning model trained to determine relative depths in endoscopic images. The relative depth map may include an array of relative depth values that may include a relative depth value corresponding to each pixel of the endoscopic image. The relative depth map may include, for example, depth values ranging from 0 to 1 where values closer to 1 are associated with shallower locations (relative to the camera) and values closer to 0 are associated with deeper locations (or vice versa). FIG. 6 provides an illustration of the values of an exemplary depth map 600 generated from an endoscopic image 602. The brighter regions of the depth map 600, such as region 604, are associated with shallower regions in the image 602 and the darker regions of the depth map, such as region 606, are associated with deeper regions in the image 602. The relative depth map may be generated based on an endoscopic image that includes one or more fiducial markers such that the relative depth map includes relative depth values for the one or more fiducial markers that are generated by the machine learning model predicting the depth of the one or more fiducial markers relative to other portions of the scene captured in the endoscopic image.

At step 504, the relative depth values of the relative depth map are calibrated based on the position of at least one fiducial marker in the endoscopic image determined at step 406 of method 400 to generate a calibrated depth map. The position of the fiducial marker determined at step 406 can include a depth (e.g., position in the z-direction of a camera-based reference frame) of the fiducial marker or a feature of the fiducial marker. The relative depth of the fiducial marker or a feature of the fiducial marker is determined from the relative depth map. A calibration factor can be determined for calibrating the relative depth of the fiducial marker to the actual depth of the fiducial marker determined in step 406 based on the relative locations of features of the fiducial marker and the predetermined fiducial marker information that may include the known distances between the features of the fiducial marker. This calibration factor can be applied to the relative depth values of the relative depth map to generate a calibrated depth map that has calibrated depth values for each location in the endoscopic image. In other words, the known size and shape of the fiducial marker is used to calibrate the depths of the relative depth map. The known size and shape of the fiducial marker can also be used to calibrate the x-axis and y-axis positions corresponding to each pixel of the endoscopic image such that the three-dimensional positions corresponding to each pixel of the endoscopic image can be determined.

At step 506, at least one normal vector is determined based on the absolute depth map determined in step 504. Step 506 may include determining a single normal vector for the location of the hole to be drilled as determined in step 408 or may include determining multiple normal vectors corresponding to various locations including the location of the hole to be drilled. For example, normal vectors may be determined for each pixel location of the endoscopic image or each pixel location in a portion of the endoscopic region, such as a portion containing the location where the hole will be drilled. A normal vector for a given location is a vector that is normal (orthogonal) to the surface at the given location. A normal vector for a given location of the surface of the tissue may be determined based on the rate of change of the depth values in the depth map at the given location. The normal vectors may be generated using convolution.

At step 508, the orientation of the surface of the tissue where the hole is to be drilled is determined based on the normal vector for that location. The normal vector may be decomposed into a plurally of three-dimensional rotations relative to a reference frame, such as a camera-based reference frame. The rotations can be, for example, a rotation about an x-axis extending in the width direction of the endoscopic image, a rotation about a y-axis extending in a height direction of the endoscopic image, and a rotation about a z-axis extending in a depth direction of the endoscopic image.

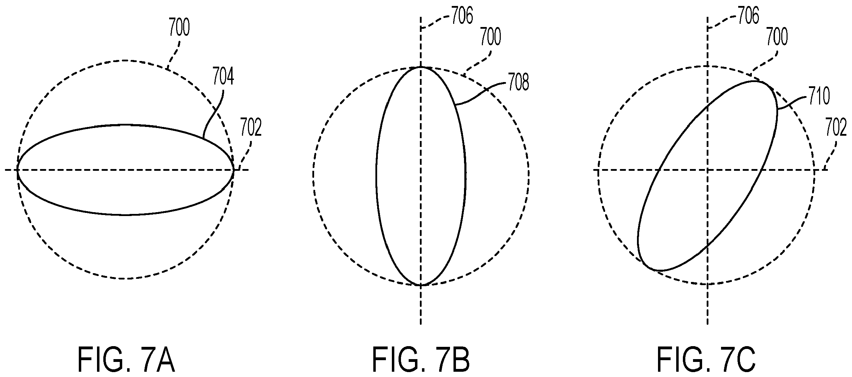

The orientation of the surface of the tissue where the hole is to be drilled can be used in step 410 of method 400 to determine the shape of a graphical indication of the hole to be drilled. For example, the graphical indication can be an oval and the shape of the oval can be determined by rotating a circle according to the three-dimensional rotations associated with the surface of the tissue determined in step 508 in combination with three-dimensional rotations associated with the position and orientation of the feature of the drill guide determined in step 408 of method 400. FIG. 7A illustrates an example of the rotation of a circle 700 about a first axis 702, resulting in a first oval 704. FIG. 7B illustrates an example of the rotation of the circle 700 about a second axis 706, resulting in a second oval 708. FIG. 7C illustrates an example of the rotation of the circle 700 about the first axis 702 and second axis 706, resulting in oval 710.

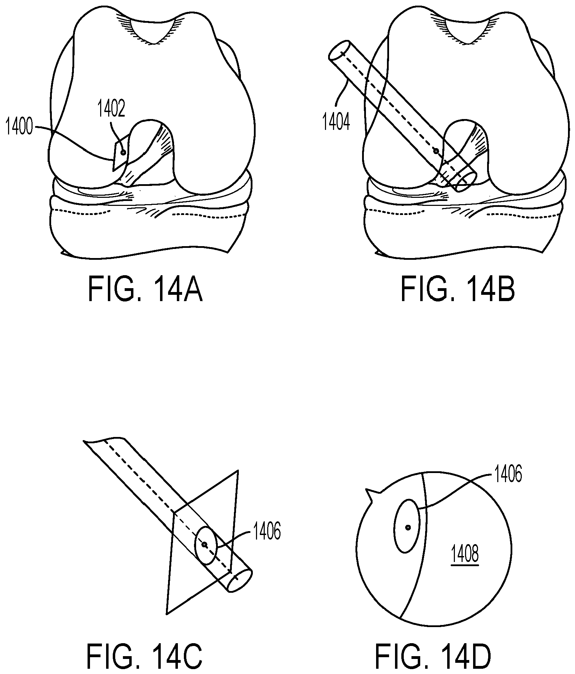

FIGS. 14A-14D illustrate an example of how the orientation of the surface of the tissue and the orientation of the hole to be drilled can be used in combination to determine the shape of a graphical indication of the hole to be drilled. As shown in FIG. 14A, a plane 1400 that is tangent to the surface of the tissue at the location 1402 where the hole is to be drilled is determined based on the normal vector determined at step 508 of method 500. As shown in FIG. 14B, the orientation of the hole to be drilled determined per step 408 of method 400 is used to orient a cylinder 1404 that has a diameter corresponding to a diameter of the hole to be drilled. As shown in FIG. 14C, the intersection of the plane 1400 and the cylinder 1404 determines the shape of the ellipse 1406 that indicates the shape of the hole to be drilled. As shown in FIG. 14D, the ellipse 1406 can be overlaid on an endoscopic image 1408 to indicate to the user the location and shape of the hole to be drilled.

The one or more graphical indications of the position and orientation of the hole to be drilled can include a graphical indication of the position of the hole relative to a location of interest in the endoscopic image. The location of interest can be, for example, a landmark of the tissue, such as an edge of the tissue and the distance from the hole to the landmark may be associated with a suitability of the location of the hole to be drilled. For example, a surgeon may desire to drill the hole such that a sufficient thickness of bone remains between the hole and an outer surface of the bone. Here, a measurement of such a thickness may be provided in the visualization based on a distance between the predicted hole to be drilled and the edge of the bone in the endoscopic image. For example, with reference to FIG. 3, graphical indication 316 indicates the distance from the perimeter of the hole to be drilled to a location of interest 318. The graphical indication can include, for example, a line drawn between the two locations, as illustrated, and/or a measurement 328 (22 mm) between the hole to be drilled and the location of interest.

The location of interest can be selected by the user, such as by the user providing an input to a touchscreen display displaying the endoscopic image at the location of the touchscreen that corresponds with the location of interest. Alternatively, the location of interest may be a landmark of the tissue that is automatically identified by the image processing system. For example, the image processing system may use a machine learning model trained to identify the landmark of the tissue. As noted above, a user may adjust the size of the hole, such as by selecting a different drill bit type and/or size. Upon such an adjustment, the graphical indication of the distance from the hole to be drilled to the location of interest may be automatically updated.

Once the location of interest in the endoscopic image is identified (either manually or automatically), the position of the location of interest relative to the endoscopic image may be translated into a three-dimensional position, such as by using the calibrated depth map determined in step 504 of method 500. This three-dimensional position of the location of interest may be compared to the three-dimensional position of the hole to be drilled, as determined at step 408, to determine the distance between the two locations, which may then be displayed in the visualization. Optionally, multiple different locations of interest may be selected and graphical indications of the distances from the hole to be drilled to those multiple locations may be displayed in the visualization.

Optionally, a visualization can include a virtual rendering of a "safe distance ring" overlayed on the endoscopic image. Such a ring can remain centered on the distal end of the arm and can be configured to highlight or otherwise indicate a region of tissue located within a pre-defined safety margin from the current hole's position (e.g., minimum: 10mm, maximum: 30mm). As the distal end of the arm moves, the safe distance ring can move with it. Such dynamic visualization can aid the user in properly positioning the arm without requiring the user to explicitly select individual landmarks.

A visualization generated according to method 400 provides guidance to a user regarding where a hole to be drilled will be located. If the surgeon does not like the placement of the hole to be drilled, the surgeon can adjust the position and/or orientation of the drill guide and the position and/or orientation of the graphical indication of the hole to be drilled in the visualization can be automatically updated to show the new position and/or orientation of the hole to be drilled. Any graphical indications of the distance from the hole to be drilled to one or more locations of interest may be updated accordingly. With this visual guidance, the surgeon can ensure that the hole is properly positioned and oriented in the tissue, which can lead to better surgical outcomes.

With the visual guidance provided according to method 400, the proper placement of a hole in the tissue may be determined by the surgeon based on the surgeon's training and experience. In some instances, the user may plan the placement of the hole pre-operatively. For example, a surgeon may use pre-operative imaging, such as CT or MRI imaging, to plan the ideal placement and orientation of a hole in the tissue. The pre-operative planning can also be achieved via a digital twin technology that simulates a surgical environment using an accurate digital twin of the patient. With the help of a digital twin, the user can practice hole drilling pre-operatively and establish a plan for the optimal location of the hole. The plan may include, for example, one or more distances between the planned hole and one or more landmarks that may be observable in endoscopic images during the surgical procedure. During the surgery, a visualization generated according to method 400 may display measurements to the landmarks (e.g., selected manually or automatically identified by the image processing system), and the surgeon may adjust the position of the drill guide until the measurements displayed in the visualization match, or otherwise correspond to, the measurements from a pre-operative plan.

Alternatively, the image processing system may be configured to generate visual guidance of a hole to be drilled based on a pre-operative plan, instead of, or in addition to, based on the placement of a drill guide. With such guidance, the surgeon could move the drill guide to the position and orientation indicated by the visual guidance generated from the pre-operative plan.

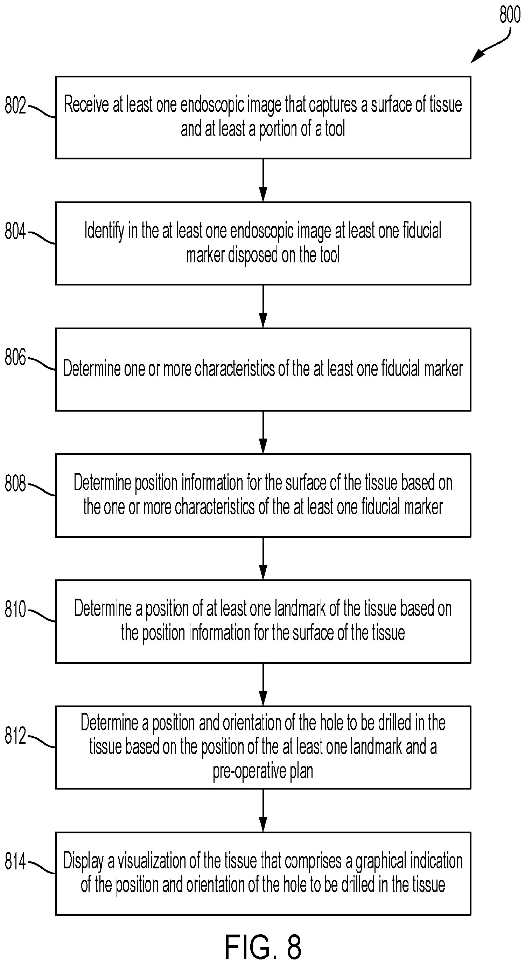

FIG. 8 illustrates an exemplary method 800 for generating and displaying a visualization that includes a graphical indication of the position and orientation of a hole to be drilled in the tissue that is generated based on a pre-operative plan. Method 800 can be performed by an image processing system, such as image processing system 116 of system 100 of FIG. 1.

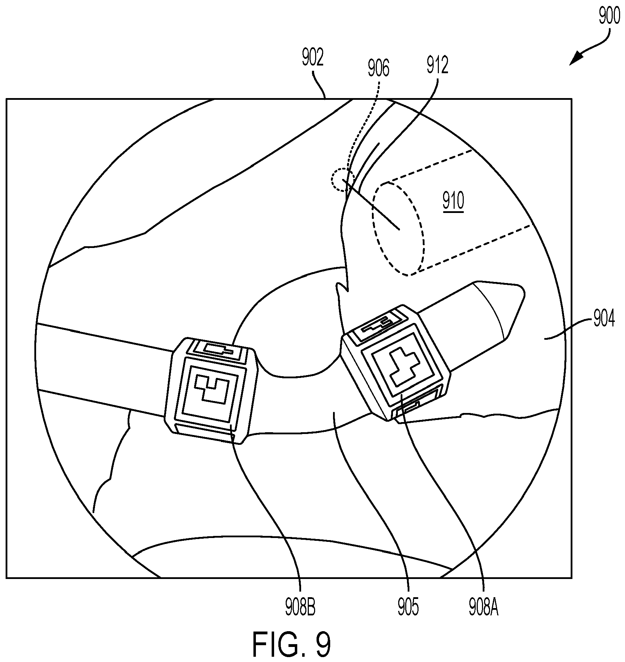

At step 802, at least one endoscopic image is received at an image processing system. The endoscopic image(s) capture a surface of tissue of interest within a surgical cavity, which may be, for example, bony anatomy. The tissue of interest may be tissue into which a hole is to be drilled based on a pre-operative plan. The endoscopic image(s) may also capture a distal portion of a tool that is located in the surgical cavity. The endoscopic imager and the tool can be pre-inserted prior to start of method 800. The tool may be a drill guide, such as drill guide 122 of FIG. 1, or may be a different type of tool, such as a pointer tool. The tool can be a hand-held tool that extends into a surgical cavity. The tool includes one or more fiducial markers and at least one of the one or more fiducial markers is captured in the at least one endoscopic image. FIG. 9 illustrates an example of an endoscopic image 902 that captures tissue of interest 904 into which a hole is to be drilled and a tool 905 that includes one or more fiducial markers 908A, 908B.

At step 804, at least one fiducial marker of the tool is identified in the at least one endoscopic image. For example, one or more of fiducial markers 908A and 908B may be identified in the endoscopic image 902. A fiducial marker can be identified by detecting one or more visual patterns of the at least one fiducial marker and matching the one or more visual patterns to at least one fiducial marker pattern in a pre-defined list of fiducial marker patterns, as discussed above with respect to step 404 of method 400. The at least one fiducial marker need only appear somewhere in the at least one endoscopic image and is not required to be a fixed relationship with the tissue (e.g., it need not be anchored to the tissue). For example, as noted above, the at least one fiducial marker can be disposed on a hand-held tool.

At step 806, one or more characteristics of the at least one fiducial marker are determined. The one or more characteristics can include, for example, a position and/or an orientation of the fiducial marker relative to a suitable reference frame, such as a camera-based reference frame. The position can include a position in one or more dimensions relative to the reference frame. The position can be, for example, a depth of the fiducial marker relative to the camera-based reference frame. The one or more characteristics of the fiducial marker can be determined by comparing the fiducial marker in the endoscopic image to predetermined information regarding the fiducial marker stored in a memory of the image processing system, in similar fashion to step 406 of method 400.

At step 808, position information for a surface of the tissue in the endoscopic image is determined. The position information may be, for example, three-dimensional positions of each pixel of the endoscopic image relative to a reference frame, such as a camera-based reference frame. The position information may be determined using one or more steps of method 500. For example, step 502 can be performed to generate a relative depth map, which can then be calibrated in step 504 using the one or more characteristics of the fiducial marker determined in step 806. For example, the depth of the fiducial marker determined in step 806 can be used to calibrate the relative depths of the relative depth map to generate absolute depth values in step 504. The one or more characteristics of the fiducial marker can also be used to calibrate the x- and y- locations of the pixels, which in combination with the depth information provides the three-dimensional position for each pixel in the endoscopic image.

At step 810, a position of at least one landmark of the tissue in the endoscopic image is determined. A landmark in the endoscopic image may be manually selected by a user, such as by the user selecting a location in the endoscopic image corresponding to the landmark (e.g., via a touch on a touchscreen or a mouse-based selection). Additionally or alternatively, one or more landmarks may be identified automatically by the image processing system using one or more machine learning models trained to identify the landmark(s). The position of the landmark can be determined based on the position information determined in step 808. For example, the position of the landmark can be determined from the three-dimensional location of the pixel or set of pixels associated with the landmark. FIG. 9 illustrates an example of a landmark 906 (indicated by the dashed circle for clarity) identified in the endoscopic image 902. Additionally or alternatively, a landmark's position can be established by the user pointing a tool (such as a pointer tool like took 905 depicted in FIG. 9) to the desired location within the surgical site. The tool may optionally have fiducial markers (e.g., fiducial markers 908A, 908B) on its distal end to facilitate a more precise landmark placement. Once the tool is placed next to the desired location, the user may confirm the landmark by providing a trigger input (e.g., a button press, foot pedal, or a voice command). Alternatively, the user may first activate a landmark placement mode and then start moving the tool within the surgical site. The movement can be accompanied by a virtual mark in the field of view following the tool and indicating the tentative landmark position. Once the user is satisfied with the landmark's placement, the user can confirm the placement using the trigger input.

At step 812, a position and orientation in the endoscopic image of a hole to be drilled in the tissue is determined based on the position of the at least one landmark determined in step 810 and a pre-operative plan. The pre-operative plan may include a planned position and orientation of the hole to be drilled relative to the at least one landmark. This information may be used in combination with the landmark position determined in step 810 to determine the pixel location in the endoscopic image where the hole to be drilled should be located. The orientation in the pre-operative plan can be an orientation relative to an orientation of the surface of the tissue where the hole is to be drilled. The orientation of the tissue where the hole is to be drilled can be determined by determining the surface normal vector according to step 506 of method 500 for the pixel location corresponding to the position of the hole to be drilled (e.g., the pixel location of the center of the hole to be drilled) specified in the pre-operative plan. The orientation information from the pre-operative plan can then be applied to the orientation of the tissue where the hole is to be drilled to determine the orientation of the hole to be drilled.

At step 814, a visualization of the tissue that comprises a graphical indication of the position and orientation of the hole to be drilled in the tissue is displayed. The graphical indication can be positioned and oriented in the visualization based on the position and orientation of the hole to be drilled determined in step 812. The visualization can include an endoscopic image with one or more graphical indications indicating the planned position and orientation of the hole to be drilled overlayed on the endoscopic image. For example, with reference to FIG. 9, a visualization 900 can include a graphical indication 910 of the hole to be drilled overlayed on the endoscopic image 902. The graphical indication 910 may be positioned at a planned distance 912 from the landmark 906 that is defined in the pre-operative plan. The graphical indication 910 may be oriented according to the planned orientation from the pre-operative plan. The visualization displayed at step 814 may guide a surgeon in positioning a drill guide for drilling the hole. For example, the surgeon can insert a drill guide into the surgical cavity and adjust the position of the drill guide until it is located at the location indicated by the graphical indication. It will be appreciated that inserting the drill guide into the surgical cavity and adjusting the position of the drill guide until it is located at the location indicated by the graphical indication is not part of the method for graphically indicating a location of a hole to be drilled in tissue. Optionally, method 400 and method 800 may be used together such that a graphical indication of a hole to be drilled that is based on a current position of the drill guide is displayed in addition to a graphical indication of the planned position of the hole to be drilled. The user could adjust the position of the drill guide until the two graphical indications align.

Visualizations generated according to method 400 and/or method 800 may include endoscopic images that are still images and the visualization may include displaying the one or more graphical indications of the hole to be drilled as an overlay on the still image. Visualizations generated according to method 400 and/or method 800 may include endoscopic images that are frames of a video feed and the visualization may include displaying the one or more graphical indications of the hole to be drilled as an overlay on the video. Since the endoscopic imager may move relative to the imaged tissue over time, the image processing system may track such movement and may relocate the graphical indication in the visualization such that the graphical indication continues to be positioned at the correct position relative to the current video frame.

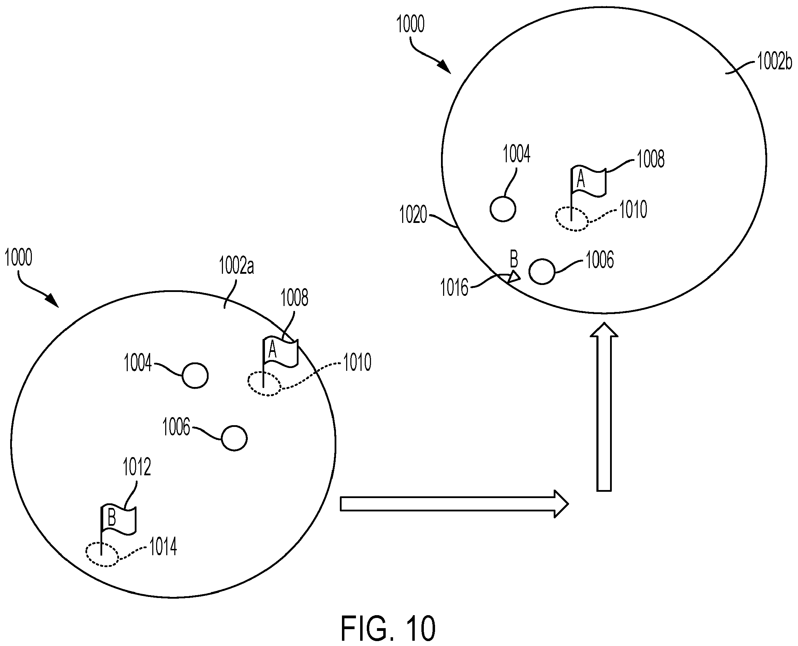

FIG. 10 illustrates an exemplary visualization of tissue of interest in which one or more graphical indications of one or more holes to be drilled in the tissue are relocated in the visualization based on movement of the endoscopic imager such that the graphical indication(s) continues to accurately indicate the position of the hole(s) to be drilled relative to the displayed scene. FIG. 10 illustrates two separate instantiations of a visualization 1000, each including a different frame 1002a and 1002b of an endoscopic video. Frame 1002a was taken earlier in time than frame 1002b. Frames 1002a and 1002b can each capture multiple features of interest 1004 and 1006 of tissue within the field of view, which as described further below, can be identified for determining camera movement. Visualization 1000 can include a flag 1008 that indicates a hole to be drilled 1010 in the tissue. The position of the flag 1008 in the visualization 1000 for frame 1002a may be determined according to method 400 of FIG. 4 or method 800 of FIG. 8. Thus, while features of interest 1004 and 1006 represent parts of the anatomy that are visible within the frame 1002a, flag 1008 is a graphical feature overlayed on the frame 1002a. One or more additional flags 1012 may be displayed for additional holes to be drilled 1014 in the tissue.

If the endoscopic imager used to generate frame 1002a remains stationary, then features of interest 1004 and 1006, as well as flag 1008, can maintain their position within the visualization 1000. However, if the endoscopic imager moves during the procedure, then the position of features of interest 1004 and 1006, as well as the hole to be drilled 1010 indicated by flag 1008, will move within the visualization, based on the direction and distance that the endoscopic imager moves. For instance, as shown in FIG. 10, frame 1002b can represent a frame that would appear if the endoscopic imager is moved to the right and up. In such an example, the position of features of interest 1004 and 1006 will appear further down and to the left in frame 1002b than in frame 1002a. Likewise, flag 1008 of frame 1002a will be moved further down and to the left in the visualization 1000.

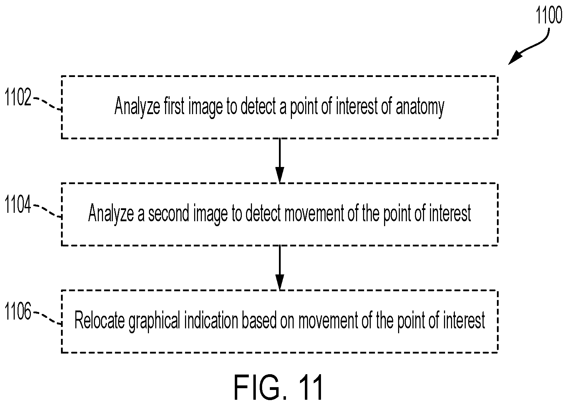

FIG. 11 illustrates an exemplary method 1100 for repositioning a graphical indication of a hole to be drilled in tissue in a visualization according to movement of an endoscopic imager. Method 1100 can be performed in combination with one or more steps of method 400 of FIG. 4 and/or method 800 of FIG. 8. For example, method 1100 can be performed for a visualization displayed in step 410 based on frames of an endoscopic video received in step 402. Method 1100 can be performed by an image processing system, such as image processing system 116 of FIG. 1.

At step 1102, at least one point of interest of the anatomy captured in a received frame (or multiple frames) of endoscopic video can be detected. The points of interest can be identified using one or more machine learning models configured to detect points of interest in images for use in computer vision applications such as the Greedily Learned Accurate Match Points (GLAM) keypoint detector, the Self-Supervised Interest Point Detector (Superpoint), Magicpoint, Unsuperpoint, or other machine learning models configured to learn correspondences in images with known homographic transformations. The machine learning model or models used to detect points of interests (i.e., keypoints) in an image can be configured to extract stable points in the image. The stable points can be used to track the motion of an endoscopic imager such as to determine the rotation and/or translation of the endoscopic imager. The rotation and/or translation of the endoscopic imager can be determined based on how those stable points move between frames of the image. The frame within which the at least one point of interest is detected may be displayed as part of a visualization that provides a graphical indication of a hole to be drilled in tissue according to method 400.

At step 1104, a frame captured after the frame used in step 1102 was captured is received and used to determine whether, and the extent to which, the endoscopic imager has moved during the time between when the first frame was captured and when the second frame was captured. The at least one point of interest detected at step 1102 can be also detected in step 1104 using one or more of the machine learning models described above with respect to step 1102. If the camera has moved between capture of the first frame and capture of the second frame, then the points of interest located in the first frame will have moved to a different position in the image frame based on the extent and direction of the motion. The movement of the interest points can be used to determine where to place the graphical indication of the hole to be drilled relative to the second frame so that the graphical indication appears stuck to the hole to be drilled and moves according to the motion of the endoscopic imager so that the graphical indication points to hole to be drilled despite the motion of the camera.

The distance and direction of the movement of the endoscopic imager can be calculated using homography, a computer vision technique that can be used to produce a motion matrix that indicates how much each pixel in the image has moved from a previous image using one or more homographic transformations. The frames of steps 1102 and 1104 can be matched using a k-nearest neighbors (KNN) algorithm with filtering that can be used to match the at least one point of interest identified in the frames. From the matched correspondences, the estimation of motion can be performed using homography.

At step 1106, the amount of motion estimated at step 1104 is used to move the graphical indication within the visualization in accordance with the estimated motion of the endoscopic imager so that the at least one graphical indication continues to point to the hole to be drilled in the tissue. If the amount of motion determined between the first captured frame and the second captured frame is such that the hole to be drilled is no longer captured in the frame (and, as a result, the graphical indication cannot be placed in the field of view), then a marker can be placed at the edge of the field of view in the visualization indicating a general direction of the hole to be drilled relative to the current view. An example of this is shown in FIG. 10 in which hole to be drilled 1014 has moved out of the field of view (i.e., is not captured in frame 1002b) and a marker 1016 is displayed at the edge of the field of view 1020 to indicate where the hole to be drilled 1014 is located relative to the currently displayed field of view.

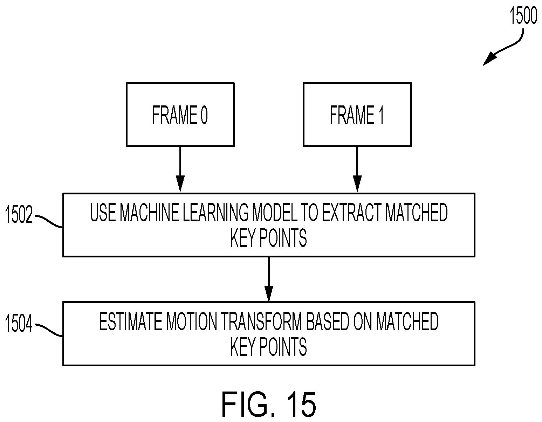

Points of interest (also referred to as key point) determination according to steps 1102 and 1104 need not be performed in discrete steps. Rather, a machine learning model may be used that analyzes a received pair of frames together to extract matched key points between the frames. The matched key points can then be used to estimate motion between the frames. FIG. 15 is a flow diagram of an exemplary method 1500 that can be performed by the image processing system to estimate motion of an endoscopic imager between frames, which can be used in place of steps 1102 and 1104 of method 1100. At step 1502, a machine learning model processes a received pair of frames-frame0 and frame1-and outputs a set of matched pairs of key points and their relative motions in three dimensions. The machine learning model used in step 1502 can be a transformer-based matching model trained using frames captured by a camera with viewpoint changes and/or illumination changes and camera positional information corresponding to the position of the camera for each frame. Examples of suitable machine learning models include Local Feature Transformer ("LoFTR"), MatchFormer, QuadTreeAttention, and TransMatcher. At step 1504, the matched key points are used to generate an estimate of motion of the endoscopic imager from frame0 to frame1. In step 1504, if the differences in relative locations of the matched key points are sufficiently large, then essential matrix techniques can be used to generate an essential matrix that relates corresponding key points in frame0 and frame1. The essential matrix can be used to derive an estimate of the translation and rotation (six degree of freedom motion) of the endoscopic imager from frame0 to frame1. If at least one of frame0 and frame1 includes a fiducial marker, then the characteristics of fiducial marker can be used to scale the essential matrix to generate a scaled estimate of motion (e.g., in millimeters). Thus, method 1500 can be used to generate an estimate of six-dimensional motion of the endoscopic imager from frame0 to frame1. This motion estimate can be used in step 1106 of method 1100 to transform the position of the hole to be drilled from its position in frame0 to its position in frame1 and update the visualization accordingly.

Other machine learning frameworks (individual machine learning models or combinations of multiple machine learning models) that do not rely upon extracting matched key points may be used for determining motion from pairs of frames. An example of such a machine learning framework is a unified self-supervised machine learning framework that estimates monocular depth and ego-motion simultaneously, such as described in a paper titled Self-Supervised Monocular Depth and Ego-Motion Estimation in Endoscopy: Appearance Flow to the Rescue (available at https://arxiv.org/pdf/2112.08122.pdf), the entire contents of which are incorporated by reference herein. This machine learning framework includes a structure module, a motion module, an appearance module, and a correspondence module to accurately reconstruct the appearance and calibrate the brightness of the frames. The structure module is a mono-depth estimator that converts a target frame into a dense depth map. The motion module functions as a six degree-of-freedom ego-motion estimator, which takes two adjacent frames as input and outputs a relative pose. The appearance module is used to predict appearance flow and align the brightness condition through a brightness calibration procedure. The correspondence module performs an automatic registration step. This machine learning framework can be trained on unlabeled endoscopic (e.g., arthroscopic) video datasets. The trained machine learning framework can then be used to estimate three-dimensional inter-frame deformation and displacement, without relying on key points or a labeled dataset.

As noted above, the output of step 1502 is the three-dimensional relative motion of the key points from frame0 to frame1. The relative motion of the key points between key frames can be combined with the motion estimate of the endoscopic imager to compute the three-dimensional positions of the key points within each frame. With suitably closely spaced key points, the orientation of a surface within a given frame can be determined from the three-dimensional positions of the key points of that surface. Thus, method 1500 can be used instead of method 500 of FIG. 5 for determining the orientation of a surface in a frame. The orientation of the surface determined according to method 1500 can then be used in step 410 of method 400 to determine the shape of a graphical indication of the hole to be drilled in, for example, frame 1.

It may not be possible to estimate motion between two frames directly, such as when the two frames are too dissimilar to match key points due to excessive motion of the endoscopic imager between the two frames. However, motion between the two frames can still be estimated by combining estimates of motion generated from one or more intermediate frames. For example, where the hole to be drilled was initially determined based on frame Fₒ, the frame of interest is Fₙ, and a frame Fₙ₋₁ was captured after frame Fₒ and before frame Fₙ, the motion between Fₒ and Fₙ can be determined by combining an estimate of motion of the endoscopic imager from frame Fₒ to frame Fₙ₋₁ with an estimate of motion of the endoscopic imager from frame Fₙ₋₁ to frame Fₙ. This can be done for any number of frames captured between frame Fₒ and frame Fₙ (e.g., Fₒ→Fₒ₊₁ + Fₒ₊₁→Fₒ₊₂ + ... + Fₙ₋₂→Fₙ₋₁ + Fₙ₋₁→Fₙ). As such, method 1500 can be performed for multiple sets of frames to generate multiple step-wise motion estimates that can be combined to determine motion from the frame in which a hole to be drilled was determined to a frame for which the position of the hole to be drilled is to be determined. This motion can then be applied to the position of one or more points in frame Fₙ to transform them to the positions they have in frame Fₒ. Thus, where the position of the hole to be drilled is known in frame Fₒ, its position in (or relative to) frame Fₙ can be determined by transforming (e.g., via matrix multiplication) its position in frame Fₒ using the sum of the motion estimates generated by method 1500.

It may not be necessary to use all frames captured between Fₒ and frame Fₙ to determine the motion of the endoscopic camera. For example, where Fₒ and frame Fₒ₊₁ are sufficiently similar due to little or no motion of the endoscopic imager, frame Fₒ₊₁ does not need to be used to generate motion estimates. Rather, the frames used to estimate motion between frame Fₒ and frame Fₙ can be a select set of key frames that have been selected based on their suitability for estimating motion. For example, where Fₒ₊₂ has been selected as a key frame, method 1500 can be applied to frames Fₒ and Fₒ₊₂ to compute the motion directly from frame Fₒ to frame Fₒ₊₂.

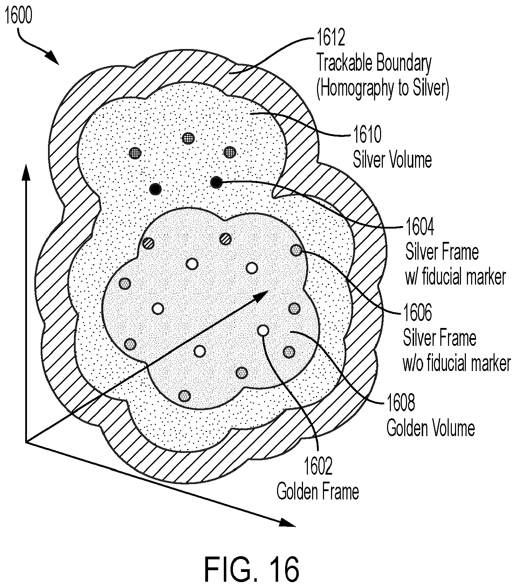

Key frames can be selected over time as frames are being captured by comparing newly captured frames to one or more previously selected key frames to determine whether there has been sufficient motion to warrant the selection of a new key frame. Over time, a key frame "cloud" can be generated that spans (with a tunable degree of density) the six degree of freedom space of endoscopic imager position and orientation. The key frame cloud can include a set of key frames stored in memory along with their corresponding six degree of freedom positions and orientations. A conceptual representation of a simplified key frame cloud representing only three degrees of freedom is illustrated in FIG. 16. Each point in the key frame cloud 1600 corresponds to a different key frame, with its location being associated with the position and orientation of the endoscopic imager (e.g., the position and orientation of the distal end of the endoscopic imager) at the time the key frame was captured. As such, each point in the key frame cloud 1600 represents a somewhat different scene from the other points in the key frame cloud 1600.

Key frame cloud 1600 may include several different types of key frames. A "golden" frame 1602 is a frame for which the position of a hole to be drilled (or any other point of interest) has been determined, such as via steps 402 to 408 of method 400 of FIG. 4 or steps 802 to 810 of method 800 of FIG. 8. There can be multiple golden frames 1602 if, for example, positions of multiple holes to be drilled have been determined. The golden volume 1608 is the portion of the six degree of freedom space for which motion can be determined from a golden frame 1602. In other words, a given frame within the golden volume 1608 and at least one golden frame 1602 can be analyzed (e.g., using method 1500) to determine the motion between the given frame and the golden frame 1602. Points outside the golden volume 1608 are too far from a nearest golden frame 1602 for motion to be determined using a golden frame 1602 directly.

The silver frames 1604 and 1606 are key frames whose position and orientation have been determined based on a golden frame 1602 or another silver frame 1604 or 1606, such as by using method 1500. Each silver frame 1604 and 1606 is sufficiently "far" from the golden frames 1602 and other silver frames 1604 and 1606 to warrant being included in the key frame cloud 1600 but not so far that the motion estimate back to a golden frame 1604 or other key frame 1604 and 1606 is unreliable or not computable. The density of the key frames (i.e., the minimum distance between key frames in six-dimensional space) can be set based on memory, computational performance, and/or motion accuracy requirements, with a sparser key frame cloud requiring less memory and less processing time than a denser key frame cloud at the potential expense of motion estimation accuracy. There may be at least two types of silver frames. Silver frames 1604 include a fiducial marker and silver frames 1606 do not include a fiducial marker. A fiducial marker in a frame can be used to "scale" the motion estimate generated in method 1500. As such, a silver frame 1606 that does not have a fiducial marker is sufficiently close to a golden frame 1602 or a silver frame 1604 that does include a fiducial marker to enable a scaled motion estimate to be generated for the silver frame 1606. The silver volume 1610 is the portion of the six degree of freedom space outside of the golden volume 1608 for which motion can be determined from a silver frame 1606 that includes a fiducial marker. The trackable boundary 1612 represents the limit of six degree of freedom space for which motion can be computed from any silver frame 1604 or 1606 using homography, such as discussed above with respect to step 1104 of method 1100.

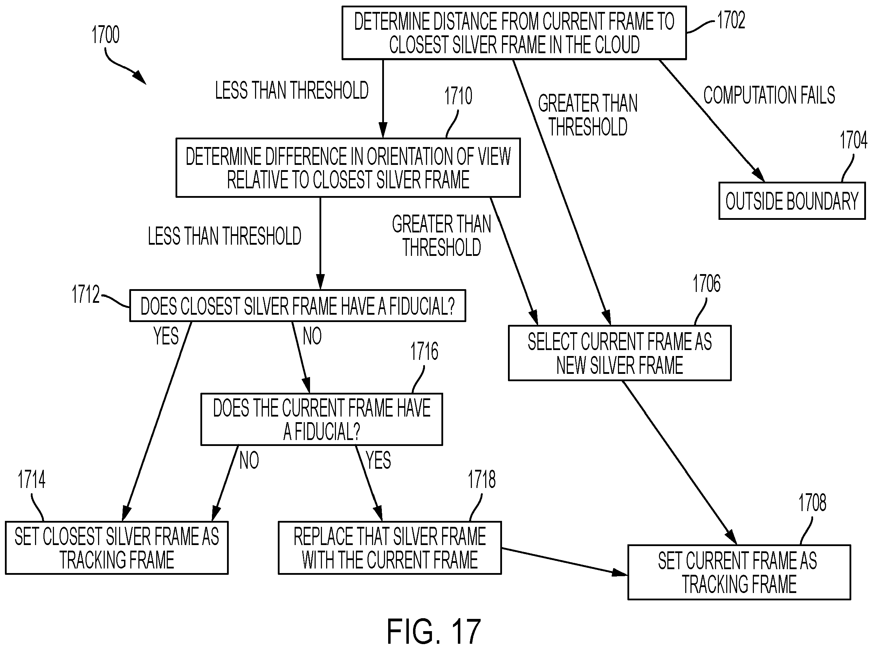

FIG. 17 is a flow diagram of an exemplary method 1700 for building a key frame cloud, such as key frame cloud 1600 of FIG. 6. Method 1700 can be performed by the image processing system periodically as frames are being captured (e.g., on each frame, every other frame, every third frame, etc.) and received by the image processing system). At step 1702, the distance from a current frame to a closest key frame in a key frame cloud is determined. The closest key frame can be a silver frame, as illustrated in FIG. 17, or can be a golden frame. The location of the current frame can be determined by estimating motion from a previous frame for which location has been determined (e.g., a tracking frame, as described below) to the current frame. This can be done by using homography and/or method 1500 to determine motion of key points and then computing an average motion across the key points of the frame. The key frame cloud is searched for a key frame that is closest in location (e.g., three-dimensional location) to the current frame's location and the distance between them is calculated. If for some reason this determination fails, such as if the current frame is too dissimilar from a previous frame due to excessive motion of the endoscopic camera (or, perhaps, excessive motion of the subject), then the current frame is determined to be outside the trackable boundary of the point cloud at step 1704 and the process ends (a warning may be provided to the user indicating excessive camera and/or subject motion).

If the distance between the current frame and closest key frame, as determined in step 1702, is greater than a threshold distance, then the current frame is selected as a new silver frame for the key frame cloud at step 1706. The threshold distance can be a physical-space distance, such as a certain number of millimeters. The current frame is then set as the tracking frame at step 1708. The tracking frame can be used for tracking motion of future frames.

If the distance between the current frame and closest key frame, as determined in step 1702, is less than the threshold distance, then a difference in orientation of the view of the current frame relative to the view of the closest key frame is determined in step 1710. If the difference in orientation is greater than a threshold (e.g., a threshold value in degrees), then method 1700 proceeds to step 1706. If the difference in orientation is less than the threshold, then at step 1712, a determination is made whether the closest key frame has a fiducial. If it does, then at step 1714, the closest key frame is set as the tracking frame. If the closest key frame does not have a fiducial, then at step 1716, a determination is made of whether the current frame has a fiducial. If it does not, then method 1700 proceeds to step 1714. If the current frame does have a fiducial, then at step 1718, the current key frame is replaced in the key frame cloud with the current frame, and the method continues to step 1708.

Method 1700 need not be performed on frames that do not have much motion relative to a previous frame. As such, method 1700 may only be performed if the motion between a current frame and a previous frame is sufficiently large. The motion between a current frame and a previous frame can be estimated using a two-dimensional motion technique, such as homography, and if the motion estimate is larger than a threshold, then method 1700 can be performed. The threshold can be, for example, a threshold number of pixels or a threshold percentage of the scope circle radius. If the estimate is below the threshold, then the two-dimensional motion estimate can be used to update the position of a hole to be drilled (or other feature of interest) for the current frame. The previous frame that the current frame is compared to can be, for example, a tracking frame determined in a previous iteration of method 1700. The motion between a current frame and a golden frame used to determine the location of a hole to be drilled (or other feature of interest) in the current frame can be determined by calculating motion from the current frame to the tracking frame and from the tracking frame back through key frames of the key frame cloud to the golden frame in a step-wise fashion. The step-wise motions can be accumulated (e.g., by adding them together).

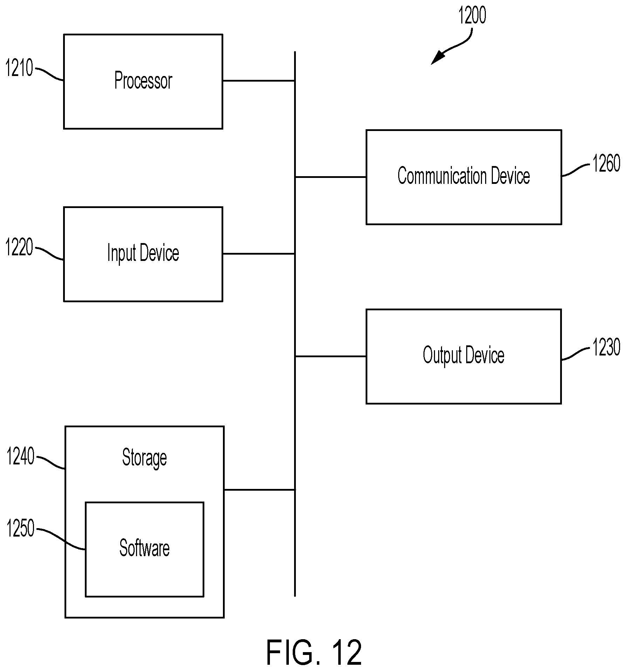

FIG. 12 illustrates an example of a computing system 1200 that can be used for one or more components of system 100 of FIG. 1, such as one or more of camera head 108, camera control unit 112, and image processing system 116. System 1200 can be a computer connected to a network, such as one or more networks of hospital, including a local area network within a room of a medical facility and a network linking different portions of the medical facility. System 1200 can be a client or a server. System 1200 can be any suitable type of processor-based system, such as a personal computer, workstation, server, handheld computing device (portable electronic device) such as a phone or tablet, or dedicated device. System 1200 can include, for example, one or more of input device 1220, output device 1230, one or more processors 1210, storage 1240, and communication device 1260. Input device 1220 and output device 1230 can generally correspond to those described above and can either be connectable or integrated with the computer.

Input device 1220 can be any suitable device that provides input, such as a touch screen, keyboard or keypad, mouse, gesture recognition component of a virtual/augmented reality system, or voice-recognition device. Output device 1230 can be or include any suitable device that provides output, such as a display, touch screen, haptics device, virtual/augmented reality display, or speaker.

Storage 1240 can be any suitable device that provides storage, such as an electrical, magnetic, or optical memory including a RAM, cache, hard drive, removable storage disk, or other non-transitory computer readable medium. Communication device 1260 can include any suitable device capable of transmitting and receiving signals over a network, such as a network interface chip or device. The components of the computing system 1200 can be connected in any suitable manner, such as via a physical bus or wirelessly.

Processor(s) 1210 can be any suitable processor or combination of processors, including any of, or any combination of, a central processing unit (CPU), field programmable gate array (FPGA), and application-specific integrated circuit (ASIC). Software 1250, which can be stored in storage 1240 and executed by one or more processors 1210, can include, for example, the programming that embodies the functionality or portions of the functionality of the present disclosure (e.g., as embodied in the devices as described above), such as programming for performing one or more steps of method 400 of FIG. 4 and/or method 800 of FIG. 8

Software 1250 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 1240, that can contain or store programming for use by or in connection with an instruction execution system, apparatus, or device.

Software 1250 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport computer readable medium can include, but is not limited to, an electronic, magnetic, optical, electromagnetic, or infrared wired or wireless propagation medium.

System 1200 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 1200 can implement any operating system suitable for operating on the network. Software 1250 can be written in any suitable programming language, such as C, C++, Java, or Python. In various embodiments, application software embodying the functionality of the present disclosure can be deployed in different configurations, such as in a client/server arrangement or through a Web browser as a Web-based application or Web service, for example.

The disclosure will now further describe the following numbered embodiments. It will be appreciated that some or all of the embodiments summarize aspects of the disclosure as provided in the detailed description and the figures. Accordingly, the embodiments are also to be read in connection with the preceding paragraphs and the appended figures, and do not limit the disclosure. The features and preferences as described hereinabove apply also to the following embodiments.
Embodiment 1. A method for visually indicating a location of a hole to be drilled in tissue comprising, at a computing system:
   receiving at least one endoscopic image that captures a surface of the tissue and at least a portion of a drill guide to be used for drilling the hole in the tissue;
   identifying in the at least one endoscopic image at least one fiducial marker disposed on the drill guide;
   determining a position and orientation of the at least one fiducial marker;
   determining a position and orientation of the hole to be drilled in the tissue based at least in part on the position and orientation of the at least one fiducial marker; and
   displaying a visualization of the tissue that comprises a graphical indication of the position and orientation of the hole to be drilled in the tissue.
Embodiment 2. The method of embodiment 1, comprising: determining an orientation of the surface of the tissue, wherein the position and orientation of the hole to be drilled in the tissue is determined based on the orientation of the surface of the tissue.
Embodiment 3. The method of embodiment 2, wherein the graphical indication comprises a graphical representation of an estimated break-through in the surface of the tissue.
Embodiment 4. The method of embodiment 3, wherein a shape of the estimated break-through in the surface of the tissue is based on the orientation of the hole to be drilled and the orientation of the surface of the tissue.
Embodiment 5. The method of embodiment 3 or embodiment 4, wherein the graphical representation of the estimated break-through in the surface of the tissue has a diameter that corresponds to a diameter of a drill bit.
Embodiment 6. The method of embodiment 5, comprising receiving a user input corresponding to a selection of a different drill bit diameter and adjusting the diameter of the estimated break-through in the surface of the tissue based on the selection of the different drill bit diameter.
Embodiment 7. The method of embodiment 5 or embodiment 6, wherein the visualization comprises a textual indication of the diameter of the drill bit.
Embodiment 8. The method of any of the preceding embodiments, wherein the visualization comprises a graphical representation of a shaft of the hole to be drilled.
Embodiment 9. The method of any of the preceding embodiments, wherein the tissue comprises bony tissue.
Embodiment 10. The method of any of the preceding embodiments, wherein the surface of the tissue in the at least one endoscopic image is opposite a surface of the tissue where a drill bit will start drilling the hole to be drilled.
Embodiment 11. The method of any of the preceding embodiments, wherein the visualization comprises an indication of a distance from the hole to be drilled to a landmark of the tissue.
Embodiment 12. The method of embodiment 11, wherein the landmark of the tissue is at a user-selected location of the tissue.
Embodiment 13. The method of embodiment 12, comprising receiving a user input relative to the displayed visualization selecting the user-selected location of the tissue.
Embodiment 14. The method of embodiment 11, wherein the landmark of the tissue is automatically identified in the at least one endoscopic image.
Embodiment 15. The method of any of embodiments 11-14, comprising receiving a user input selecting a different drill bit diameter and adjusting the indication of the distance from the hole to be drilled to the landmark of the tissue based on the different drill bit diameter.
Embodiment 16. The method of any of embodiments 2-7, or any of embodiments 8-15 when dependent on any of embodiments 2-7, wherein determining the orientation of the surface of the tissue comprises:
   generating a depth map that comprises depths at different locations of the surface of the tissue relative to a camera reference frame; and
   determining normal vectors of the surface of the tissue based on the depth map.
Embodiment 17. The method of embodiment 16, wherein generating the depth map comprises calibrating the depth map based on the position and orientation of the at least one fiducial marker.
Embodiment 18. The method of embodiment 16 or embodiment 17, wherein generating the depth map comprises predicting the depths at the different locations of the surface of the tissue using a machine learning model.
Embodiment 19. The method of any of embodiments 2-7, or any of embodiments 8-15 when dependent on any of embodiments 2-7, wherein determining the orientation of the surface of the tissue comprises:
   determining relative motions of matched key points based on the at least one endoscopic image and a previously captured endoscopic image;
   determining three-dimensional positions of key points within the at least one endoscope image based on the relative motions of matched key points; and
   determining the orientation of the surface of the tissue based on the three-dimensional positions of key points associated with the surface of the tissue.
Embodiment 20. A system comprising one or more processors, memory, and at least one program stored in the memory for execution by the one or more processors and comprising instructions that, when executed by the one or more processors, cause the system to:
   receive at least one endoscopic image that captures a surface of the tissue and at least a portion of a drill guide to be used for drilling the hole in the tissue;
   identify in the at least one endoscopic image at least one fiducial marker disposed on the drill guide;
   determine a position and orientation of the at least one fiducial marker;
   determine a position and orientation of the hole to be drilled in the tissue based on the position and orientation of the at least one fiducial marker; and
   display a visualization of the tissue that comprises a graphical indication of the position and orientation of the hole to be drilled in the tissue.
Embodiment 21. The system of embodiment 20, wherein the at least one program comprises instructions for: determining an orientation of the surface of the tissue, wherein the position and orientation of the hole to be drilled in the tissue is determined based on the orientation of the surface of the tissue.
Embodiment 22. The system of embodiment 21, wherein the graphical indication comprises a graphical representation of an estimated break-through in the surface of the tissue.
Embodiment 23. The system of embodiment 22, wherein a shape of the estimated break-through in the surface of the tissue is based on the orientation of the hole to be drilled and the orientation of the surface of the tissue.
Embodiment 24. The system of embodiment 22 or embodiment 23, wherein the graphical representation of the estimated break-through in the surface of the tissue has a diameter that corresponds to a diameter of a drill bit.
Embodiment 25. The system of embodiment 24, wherein the at least one program comprises instructions for receiving a user input corresponding to a selection of a different drill bit diameter and adjusting the diameter of the estimated break-through in the surface of the tissue based on the selection of the different drill bit diameter.
Embodiment 26. The system of embodiment 24 or embodiment 25, wherein the visualization comprises a textual indication of the diameter of the drill bit.
Embodiment 27. The system of any of embodiments 20-26, wherein the visualization comprises a graphical representation of a shaft of the hole to be drilled.
Embodiment 28. The system of any of embodiments 20-27, wherein the tissue comprises bony tissue.
Embodiment 29. The system of any of embodiments 20-28, wherein the surface of the tissue in the at least one endoscopic image is opposite a surface of the tissue where a drill bit will start drilling the hole to be drilled.
Embodiment 30. The system of any of embodiments 20-29, wherein the visualization comprises an indication of a distance from the hole to be drilled to a landmark of the tissue.
Embodiment 31. The system of embodiment 30, wherein the landmark of the tissue is at a user-selected location of the tissue.
Embodiment 32. The system of embodiment 31, wherein the at least one program comprises instructions for receiving a user input relative to the displayed visualization selecting the user-selected location of the tissue.
Embodiment 33. The system of embodiment 30, wherein the landmark of the tissue is automatically identified in the at least one endoscopic image.
Embodiment 34. The system of any of embodiments 30-33, wherein the at least one program comprises instructions for receiving a user input selecting a different drill bit diameter and adjusting the indication of the distance from the hole to be drilled to the landmark of the tissue based on the different drill bit diameter.
Embodiment 35. The system of any of embodiments 21-26, or any of embodiments 27-34 when dependent on any of embodiments 21-26, wherein determining the orientation of the surface of the tissue comprises:
   generating a depth map that comprises depths at different locations of the surface of the tissue relative to a camera reference frame; and
   determining normal vectors of the surface of the tissue based on the depth map.
Embodiment 36. The system of embodiment 35, wherein generating the depth map comprises calibrating the depth map based on the position and orientation of the at least one fiducial marker.
Embodiment 37. The system of embodiment 35 or embodiment 36, wherein generating the depth map comprises predicting the depths at the different locations of the surface of the tissue using a machine learning model.
Embodiment 38. The system of any of embodiments 21-26, or any of embodiments 27-34 when dependent on any of embodiments 21-26, wherein determining the orientation of the surface of the tissue comprises:
   determining relative motions of matched key points based on the at least one endoscopic image and a previously captured endoscopic image;
   determining three-dimensional positions of key points within the at least one endoscope image based on the relative motions of matched key points; and
   determining the orientation of the surface of the tissue based on the three-dimensional positions of key points associated with the surface of the tissue.
Embodiment 39. A method for visually indicating a location of a hole to be drilled in tissue comprising, at a computing system:
   receiving at least one endoscopic image that captures a surface of the tissue and at least a portion of a tool;
   identifying in the at least one endoscopic image at least one fiducial marker disposed on the tool;
   determining one or more characteristics of the at least one fiducial marker;
   determining position information for the surface of the tissue based on the one or more characteristics of the at least one fiducial marker;
   determining a position of at least one landmark of the tissue based on the position information for the surface of the tissue;
   determining a position and orientation of the hole to be drilled in the tissue based on the position of the at least one landmark and a pre-operative plan; and
   displaying a visualization of the tissue that comprises a graphical indication of the position and orientation of the hole to be drilled in the tissue.
Embodiment 40. The method of embodiment 39, wherein determining position information for the surface of the tissue comprises generating a depth map that comprises depths at different locations of the surface of the tissue relative to a camera reference frame and calibrating the depth map using the one or more characteristics of the at least one fiducial marker.
Embodiment 41. The method of embodiment 40, wherein determining the position of the at least one landmark comprises determining a depth of the at least one landmark based on the depth map.
Embodiment 42. The method of any of embodiments 39-41, wherein determining the position of the at least one landmark comprises automatically identifying the at least one landmark.
Embodiment 43. The method of any of embodiments 39-42, comprising, prior to determining the position of the at least one landmark, receiving a user input selecting the at least one landmark.
Embodiment 44. A system comprising one or more processors, memory, and at least one program stored in the memory for execution by the one or more processors and comprising instructions that, when executed by the one or more processors, cause the system to:
   receive at least one endoscopic image that captures a surface of the tissue and at least a portion of a tool;
   identify in the at least one endoscopic image and at least one fiducial marker disposed on the tool;
   determine one or more characteristics of the at least one fiducial marker;
   determine position information for the surface of the tissue based on the one or more characteristics of the at least one fiducial marker;
   determine a position of at least one landmark of the tissue based on the position information for the surface of the tissue;
   determine a position and orientation of the hole to be drilled in the tissue based on the position of the at least one landmark and a pre-operative plan; and
   display a visualization of the tissue that comprises a graphical indication of the position and orientation of the hole to be drilled in the tissue.
Embodiment 45. The system of embodiment 44, wherein determining position information for the surface of the tissue comprises generating a depth map that comprises depths at different locations of the surface of the tissue relative to a camera reference frame and calibrating the depth map using the one or more characteristics of the at least one fiducial marker.
Embodiment 46. The system of embodiment 45, wherein determining the position of the at least one landmark comprises determining a depth of the at least one landmark based on the depth map.
Embodiment 47. The system of any of embodiments 44-46, wherein determining the position of the at least one landmark comprises automatically identifying the at least one landmark.
Embodiment 48. The system of any of embodiments 44-47, wherein the at least one program comprises instructions for, prior to determining the position of the at least one landmark, receiving a user input selecting the at least one landmark.
Embodiment 49. A non-transitory computer readable storage medium storing instructions for execution by a computing system to cause the computing system to perform the method of any of embodiments 1-19 and 39-43.
Embodiment 50. A computer program product comprising software code portions for execution by a computing system to cause the computing system to perform the method of any of embodiments 1-19 and 39-43.

The foregoing description, for the purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the techniques and their practical applications. Others skilled in the art are thereby enabled to best utilize the techniques and various embodiments with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A system comprising one or more processors, memory, and at least one program stored in the memory for execution by the one or more processors and comprising instructions that, when executed by the one or more processors, cause the system to:
receive at least one endoscopic image that captures a surface of the tissue and at least a portion of a drill guide to be used for drilling the hole in the tissue;
identify in the at least one endoscopic image at least one fiducial marker disposed on the drill guide;
determine a position and orientation of the at least one fiducial marker;
determine a position and orientation of the hole to be drilled in the tissue based on the position and orientation of the at least one fiducial marker; and
display a visualization of the tissue that comprises a graphical indication of the position and orientation of the hole to be drilled in the tissue.

2. The system of claim 1, wherein the at least one program comprises instructions for: determining an orientation of the surface of the tissue, wherein the position and orientation of the hole to be drilled in the tissue is determined based on the orientation of the surface of the tissue, wherein optionally the graphical indication comprises a graphical representation of an estimated break-through in the surface of the tissue, wherein further optionally a) a shape of the estimated break-through in the surface of the tissue is based on the orientation of the hole to be drilled and the orientation of the surface of the tissue, and/or b) the graphical representation of the estimated break-through in the surface of the tissue has a diameter that corresponds to a diameter of a drill bit, wherein optionally i) the at least one program comprises instructions for receiving a user input corresponding to a selection of a different drill bit diameter and adjusting the diameter of the estimated break-through in the surface of the tissue based on the selection of the different drill bit diameter, and/or ii) the visualization comprises a textual indication of the diameter of the drill bit.

3. The system of claim 1 or 2, wherein the visualization comprises a graphical representation of a shaft of the hole to be drilled, and/or an indication of a distance from the hole to be drilled to a landmark of the tissue.

4. The system of claim 3, wherein the landmark of the tissue is at a user-selected location of the tissue, wherein optionally the at least one program comprises instructions for receiving a user input relative to the displayed visualization selecting the user-selected location of the tissue, and/or wherein the landmark of the tissue is automatically identified in the at least one endoscopic image.

5. The system of claim 3 or 4, wherein the at least one program comprises instructions for receiving a user input selecting a different drill bit diameter and adjusting the indication of the distance from the hole to be drilled to the landmark of the tissue based on the different drill bit diameter.

6. The system of claim 2 or any of claims 3-5 when dependent on claim 2, wherein determining the orientation of the surface of the tissue comprises:
generating a depth map that comprises depths at different locations of the surface of the tissue relative to a camera reference frame; and
determining normal vectors of the surface of the tissue based on the depth map.

7. The system of claim 6, wherein generating the depth map comprises calibrating the depth map based on the position and orientation of the at least one fiducial marker, and/or predicting the depths at the different locations of the surface of the tissue using a machine learning model.

8. The system of claim 2 or any of claims 3-7 when dependent on claims 2, wherein determining the orientation of the surface of the tissue comprises:
determining relative motions of matched key points based on the at least one endoscopic image and a previously captured endoscopic image;
determining three-dimensional positions of key points within the at least one endoscope image based on the relative motions of matched key points; and
determining the orientation of the surface of the tissue based on the three-dimensional positions of key points associated with the surface of the tissue.

9. A method for visually indicating a location of a hole to be drilled in tissue comprising, at a computing system:
receiving at least one endoscopic image that captures a surface of the tissue and at least a portion of a drill guide to be used for drilling the hole in the tissue;
identifying in the at least one endoscopic image at least one fiducial marker disposed on the drill guide;
determining a position and orientation of the at least one fiducial marker;
determining a position and orientation of the hole to be drilled in the tissue based at least in part on the position and orientation of the at least one fiducial marker; and
displaying a visualization of the tissue that comprises a graphical indication of the position and orientation of the hole to be drilled in the tissue.

10. The method of claim 1, comprising: determining an orientation of the surface of the tissue, wherein the position and orientation of the hole to be drilled in the tissue is determined based on the orientation of the surface of the tissue; wherein optionally the graphical indication comprises a graphical representation of an estimated break-through in the surface of the tissue, wherein further optionally a) a shape of the estimated break-through in the surface of the tissue is based on the orientation of the hole to be drilled and the orientation of the surface of the tissue, and/or b) the graphical representation of the estimated break-through in the surface of the tissue has a diameter that corresponds to a diameter of a drill bit; wherein optionally i) the method comprises receiving a user input corresponding to a selection of a different drill bit diameter and adjusting the diameter of the estimated break-through in the surface of the tissue based on the selection of the different drill bit diameter, and/or ii) the visualization comprises a textual indication of the diameter of the drill bit.

11. The method of claim 9 or 10, wherein the tissue comprises bony tissue.

12. The method of any of claims 9-11, wherein the surface of the tissue in the at least one endoscopic image is opposite a surface of the tissue where a drill bit will start drilling the hole to be drilled.

13. The method of claim 9 or 12, wherein the visualization comprises a graphical representation of a shaft of the hole to be drilled, and/or an indication of a distance from the hole to be drilled to a landmark of the tissue.

14. The method of claim 13, wherein the landmark of the tissue is at a user-selected location of the tissue, the method optionally comprising receiving a user input relative to the displayed visualization selecting the user-selected location of the tissue, and/or wherein the landmark of the tissue is automatically identified in the at least one endoscopic image.

15. A computer program product comprising software code portions for execution by a computing system to cause the computing system to perform the method of any of claims 9-14.
